# EUROPEAN PATENT SPECIFICATION

(11) **EP 4 272 721 B1**
(45) Date of publication and mention of the grant of the patent: **18.11.2026**
(21) Application number: 21914911.9
(22) Date of filing: 04.01.2021
(51) Int. Cl.: A61J 1/05, B65D 47/18, A61F 9/00

(54) **LIQUID DISCHARGE DEVICE AND DOSING DEVICE**
FLÜSSIGKEITSAUSSTOSSVORRICHTUNG UND DOSIERVORRICHTUNG
DISPOSITIF D'ÉVACUATION DE LIQUIDE ET DISPOSITIF DE DOSAGE

(43) Date of publication of application: 08.11.2023
(73) Proprietor: Kusaoke, Daiki, Kusatsu-shi, Shiga, 525-0050 (JP); Taisei Kako Co., Ltd., Osaka 531-0072 (JP)
(72) Inventor: KUWANO, Mitsuaki, Souraku-gun, Kyoto 619-0232 (JP)
(74) Representative: Hoffmann Eitle
(86) International application number: PCT/JP2021/000021
(87) International publication number: WO 2022/145049

(56) References cited:
- WO-A1-2007/111256
- WO-A1-2010/007961
- US-A1- 2009 318 883
- US-A1- 2011 106 024
- US-A1- 2018 272 364

## Description

### TECHNICAL FIELD

The present invention relates to a liquid discharge device that discharges a small amount of liquid. Further, the present invention relates to a dosing device that can be suitably used in a case where a small amount of a liquid medicine such as eye drops are administered to an eyeball or the like of a patient.

### BACKGROUND ART

In recent years, with the aging of patients, the number of patients who cannot administer the eye drops while looking upward or who cannot accurately drop the eye drops onto the surface of the eyeball is increasing. In addition, a patient with rheumatism or a patient with poor grip strength cannot press an eye-drop container by himself or herself or cannot open the eye-drop container.

In addition, it is said that the maximum amount of eye drops that can be held on the surface of the human eyeball is about 7 µL. Therefore, in a case where as much as 25 to 50 µL of eye drops are dropwise administered as in the related art, not only an excessive amount of eye drops inevitably flow out of the eye to cause the medicine not to be effectively utilized, but also there is a concern that the eye drops are absorbed by tissues around the eye and side effects associated with migration to the whole body are caused.

Therefore, an eyeglass-type ophthalmic device that administers a small amount of drug to the eye has been proposed (Patent Document 1 and Patent Document 2).

In Patent Document 1, a thermal jet type dispenser is employed as liquid injection means (refer to Figs. 5 and 6 of Patent Document 1), and in Patent Document 2, a piezo jet type dispenser or a thermal jet type dispenser is employed.

### PRIOR ART DOCUMENTS

### PATENT DOCUMENTS

Patent Document 1: WO 1994/003135 A
Patent Document 2: JP 2005-21701 A

Further related art may be found in US 2011/106024 A1, which describes a container with squeezing nozzle and lever, and in US 2009/0318883 A1, which is directed to a liquid delivery container.

### DISCLOSURE OF INVENTION

### TECHNICAL PROBLEM

However, the flying distance of the liquid medicine by the thermal jet type or the piezo jet type as in Patent Documents 1 and 2 is only about several millimeters in the horizontal direction, and it is difficult to perform accurate administration unless the discharge port is brought close to the eyeball. Therefore, there is a problem that it is difficult to perform the medication in a state of facing the front, and the accuracy of the administration is poor in a case where the medication is directly performed by his/her hand or in a case where the medication is performed by a third party such as a doctor. In addition, the device and the like are complicated, and thus, there is a problem that the product cost could increase.

Therefore, an object of the present invention is to provide a liquid discharge device capable of discharging a predetermined small amount of liquid. In addition, another object of the present invention is to provide a dosing device capable of easily administering a predetermined small amount of a liquid medicine even by a person who is difficult to administer a liquid medicine by himself or herself, a doctor, or the like.

### SOLUTION TO PROBLEM

A liquid discharge device according to the present invention is defined by the appended independent claim 1. A dosing device according to the present invention is defined by the appended claim 12. The dependent claims describe optional features and distinct embodiments. An aspect of the present invention for resolving the above-described problem is a liquid discharge device including: a liquid storage portion; a tube member; a striking portion; an operation portion; and a discharge port capable of discharging liquid, wherein the tube member has an end portion communicating with an inside of the liquid storage portion, the tube member delivering the liquid from the liquid storage portion to a discharge port side, wherein the tube member has an elastic region elastically deformable at least partly in a longitudinal direction, and wherein when the operation portion is operated, at least a part of the elastic region of the tube member is struck with an impact force by the striking portion to allow the liquid in the tube member to be pushed out and discharged from the discharge port.

The term "strike with an impact force or strike" as used herein means to strike by applying an impact force.

The term "liquid" as used herein refers to one having fluidity, and includes a liquid medicine, oil, water, an organic solvent, an aqueous solution, an ionic liquid, and the like.

In the claimed invention, the tube member delivers the liquid from the liquid storage portion to the discharge port side by capillarity.

In a preferable aspect, an entire region of the tube member constitutes the elastic region.

In a preferable aspect, the elastic region is formed of at least one elastic material selected from the group consisting of polyethylene, polypropylene, silicone, fluororubber, polytetrafluoroethylene, nylon, polyethylene terephthalate, polybutylene terephthalate, polyethylene naphthalate, natural rubber, and synthetic rubber.

In a preferable aspect, a maximum inner diameter of the elastic region in the tube member is 0.1 mm or more and 2 mm or less.

The term "maximum inner diameter" as used herein refers to the smallest enclosing circle diameter of the internal space of the tube member in the cross section orthogonal to the extension direction of the tube member, and refers to the diameter of the smallest circle including the entire internal space.

In a preferable aspect, a discharge amount of the liquid from the discharge port when the operation portion is operated once is 0.01 µL or more and 100 µL or less.

In a preferable aspect, an area of the tube member struck by the striking portion is a range of 1 mm or more and 30 mm or less in a longitudinal direction.

In a preferable aspect, when the operation portion is operated with the discharge port facing a horizontal direction, the liquid is discharged from the discharge port substantially linearly at least in a range of more than 0 cm and 0.5 cm or less.

The term "substantially linearly" as used herein includes not only a case of being completely linear but also a case of being slightly curved to an extent of being regarded as almost linear due to the influence of gravity or the like. Specifically, the angle of the straight line connecting the start point (liquid discharge port) and the end point is 5 degrees or less, preferably 3 degrees or less, and more preferably 1 degree or less.

In a preferable aspect, the liquid discharge device further includes: a main body portion; and a liquid cartridge, wherein the main body portion includes the striking portion and the operation portion, and wherein the liquid cartridge includes the liquid storage portion and the tube member, the liquid cartridge being attachable to and detachable from the main body portion.

In a preferable aspect, the liquid discharge device further includes: a case portion accommodating at least a part of the tube member so as to incorporate the discharge port therein; and a closing portion configured to move or change a posture relative to the case portion, following a movement of the operation portion, the case portion having a liquid passage hole at a position facing the discharge port, wherein the liquid discharge device includes: a closed state where the closing portion closes the liquid passage hole; and an open state where the closing portion opens the liquid passage hole, the closed state and the open state being changeable when the operation portion is operated to move or to change a posture of the closing portion.

In a preferable aspect, the liquid discharge device further includes a case member; and a striking member having the striking portion, wherein the striking member has a connection portion supported in a cantilever manner by the case member, wherein the elastic region of the tube member is at a position higher than the connection portion, and wherein when the operation portion is operated, the striking portion is elastically deformed to allow the striking portion to strike at least a part of the elastic region by a restoring force.

An aspect of the present invention is a liquid discharge device including: a liquid storage portion; a tube member; a striking portion; an operation portion; and a discharge port, wherein the tube member has a first end portion immersed in liquid inside the liquid storage portion, and a second end communicating with an outside via the discharge port, wherein the tube member has an elastic region at an intermediate portion in a longitudinal direction, the elastic region being elastically deformable and having a maximum inner diameter of 0.1 mm or more and 2 mm or less, and wherein when the operation portion is operated, at least a part of the elastic region of the tube member is struck with an impact force by the striking portion to allow the liquid inside the liquid storage portion to be pushed out and to be discharged from the discharge port.

The term "intermediate portion" as used herein refers to a portion between opposing end portions.

An aspect of the present invention is a dosing device using the liquid discharge device described above, wherein the liquid is a liquid medicine.

### EFFECT OF INVENTION

With the liquid discharge device of the present invention, it is possible to discharge a predetermined small amount of liquid.

With the dosing device of the present invention, it is possible to easily perform administration even by a person who is difficult to perform administration or by a doctor or the like at the time of medical examination.

### BRIEF DESCRIPTION OF DRAWINGS

Fig. 1 is a side view illustrating a general usage condition of a dosing device according to a first embodiment of the present invention.
Fig. 2 is a perspective view of the dosing device of Fig. 1.
Fig. 3 is an exploded perspective view of the dosing device of Fig. 2.
Fig. 4 is a perspective view of a second case portion of Fig. 3 as viewed from a direction different from that of Fig. 3.
Fig. 5 is a perspective view of a striking member of Fig. 3 as viewed from a direction different from that of Fig. 3.
Fig. 6 is a cross-sectional perspective view of a liquid medicine unit of Fig. 3.
Fig. 7 is a perspective view of the liquid medicine unit of Fig. 3 as viewed from a direction different from that of Fig. 3.
Fig. 8 is a bottom view of the liquid medicine unit of Fig. 3.
Fig. 9 is a side view of the dosing device in a state where the second case portion of Fig. 2 is removed.
Figs. 10A to 10C are explanatory views of the dosing device in a case where a liquid medicine is administered using the dosing device of Fig. 1, wherein Fig. 10A is a side view illustrating a state where an operation portion is operated, Fig. 10B is a side view illustrating a state where the operation portion returns by a energizing force of an energizing member, and Fig. 10C is a side view illustrating a state where a part of a tube member is struck by a striking portion. Figs. 10A to 10C are all drawn with a second case portion removed.
Figs. 11A to 11D are cross-sectional views illustrating a flow of a liquid medicine near the tube member in a case where the liquid medicine is administered using the dosing device of Fig. 10, and the transition is made in the order of Fig. 11A to Fig. 11D. The liquid medicine is indicated by dots.
Figs. 12A and 12B are perspective views of a dosing device according to a second embodiment of the present invention, wherein Fig. 12A is a perspective view of a closed state before a discharge operation, and Fig. 12B is a perspective view of an open state at the time of discharge.
Fig. 13 is an exploded perspective view of the dosing device of Fig. 12 as viewed from a direction different from that of Fig. 12.
Fig. 14 is an exploded perspective view of a main body portion of Fig. 13 as viewed from the same direction of Fig. 12.
Fig. 15 is a cross-sectional view taken along line A-A of the dosing device of Fig. 12A.
Fig. 16 is a perspective view of a case member of Fig. 14.
Fig. 17 is a perspective view of a case portion of Fig. 14.
Fig. 18 is a perspective view of the operation member of Fig. 14.
Fig. 19 is a perspective view of a liquid medicine cartridge of Fig. 13 as viewed from a direction different from that of Fig. 13.
Figs. 20A and 20B are explanatory views of an operation procedure of the dosing device of Fig. 12, and Figs. 20A and 20B are cross-sectional views illustrating a positional relationship in each procedure.
Figs. 21A and 21B are explanatory views of an operation procedure of the dosing device subsequent to Fig. 20B, and Figs. 21A and 21B are cross-sectional views illustrating a positional relationship in each procedure.
Figs. 22A and 22B are explanatory views of an operation procedure of the dosing device subsequent to Fig. 21B, and Figs. 22A and 22B are cross-sectional views illustrating a positional relationship in each procedure.
Figs. 23A and 23B are perspective views of a dosing device according to a third embodiment of the present invention, wherein Fig. 23A is a perspective view of a closed state before a discharge operation, and Fig. 23B is a perspective view of an open state at the time of discharge.
Fig. 24 is an exploded perspective view of the dosing device of Fig. 23 as viewed from a direction different from that of Fig. 23.
Fig. 25 is an explanatory view of the vicinity of a tube member according to another embodiment of the present invention.
Fig. 26 is a cross-sectional perspective view of a tube member according to another embodiment of the present invention.
Fig. 27 is a cross-sectional perspective view of a liquid medicine unit according to another embodiment of the present invention.

### BEST MODE FOR CARRYING OUT THE INVENTION

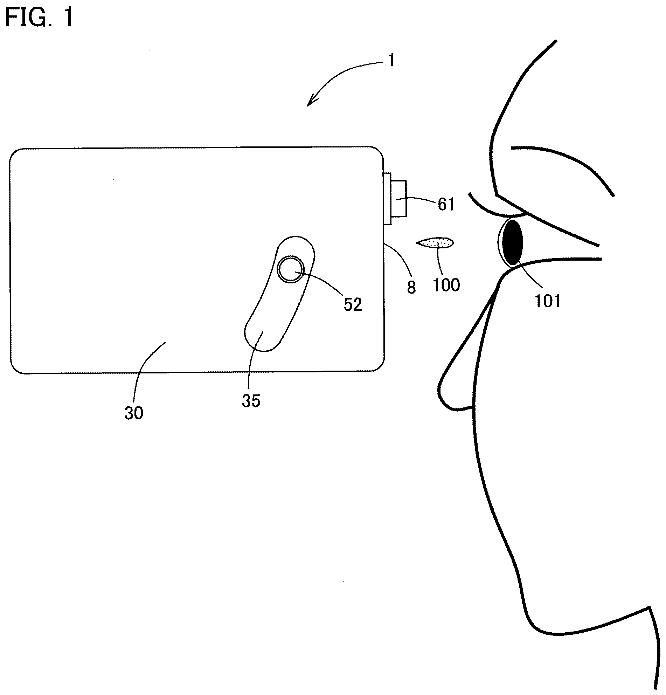

Hereinafter, embodiments of the present invention will be described in detail. In the following description, unless otherwise specified, an eyeball 101 side of a patient is defined as the front side, and the distal side as viewed from the patient is defined as the rear side, based on a posture (vertical posture) in Fig. 1.

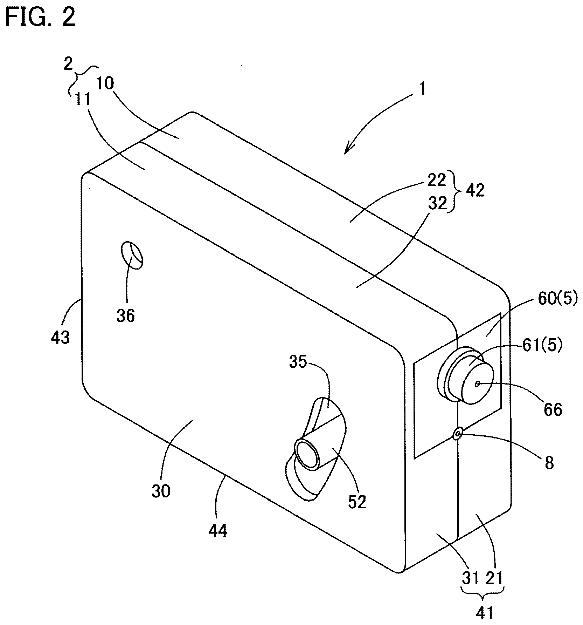

A dosing device 1 (liquid discharge device) of the first embodiment of the present invention includes a discharge port 8 on an end surface as illustrated in Figs. 1 and 2, and administers a droplet-shaped liquid medicine 100 (liquid) from the discharge port 8 to the surface of the eyeball 101 of the patient.

One of the features of the dosing device 1 is that, as illustrated in Fig. 1, the dosing device 1 can discharge the liquid medicine 100 from the discharge port 8 in the horizontal direction in a state where the discharge port 8 is in a horizontal posture facing the horizontal direction, and can administer the liquid medicine 100 without the patient facing upward, that is, regardless of the patient's posture.

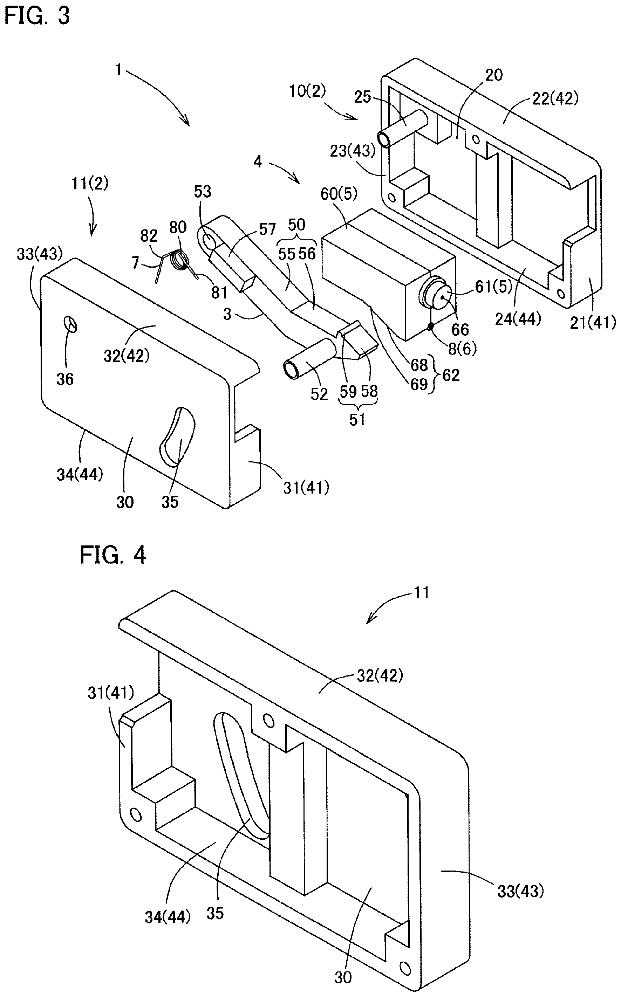

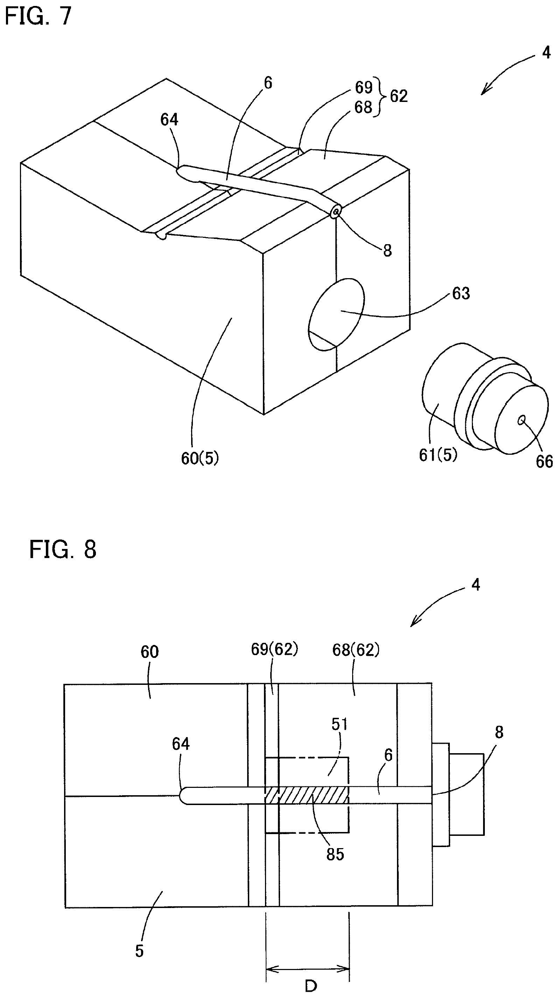

The dosing device 1 includes, as main components, a case member 2, a striking member 3, a liquid medicine unit 4, an energizing member 7, and the discharge port 8 as illustrated in Figs. 3 and 7.

The liquid medicine unit 4 mainly includes a liquid medicine storage portion 5 (liquid storage portion) and a tube member 6.

As illustrated in Fig. 3, the case member 2 includes a first case portion 10, and a second case portion 11.

The first case portion 10 includes a main body wall portion 20, side wall portions 21 to 24, and a shaft portion 25.

The main body wall portion 20 is a substantially quadrangular wall portion, and the side wall portions 21 to 24 are standing wall portions standing from each side of the main body wall portion 20.

The shaft portion 25 is a rod-shaped part erected with respect to the main body wall portion 20, and is a part forming a rotation axis of the striking member 3 at the time of assembly. The shaft portion 25 is also a fixing portion for fixing the energizing member 7.

As illustrated in Fig. 4, the second case portion 11 includes a main body wall portion 30, side wall portions 31 to 34, a guide groove 35, and a shaft fixing hole 36.

The main body wall portion 30 is a substantially quadrangular wall portion, and the side wall portions 31 to 34 are standing wall portions standing from each side of the main body wall portion 30.

The guide groove 35 is a groove for guiding the striking member 3, and is a through-groove that is provided in the main body wall portion 30, and penetrates the main body wall portion 30 in a thickness direction. The guide groove 35 extends in an arc shape in a circumferential direction around the shaft fixing hole 36 when the main body wall portion 30 is viewed from the front.

As illustrated in Fig. 3, the shaft fixing hole 36 is a bearing hole for receiving an end portion of the shaft portion 25 of the first case portion 10, and is a through-hole penetrating the main body wall portion 30 in the thickness direction.

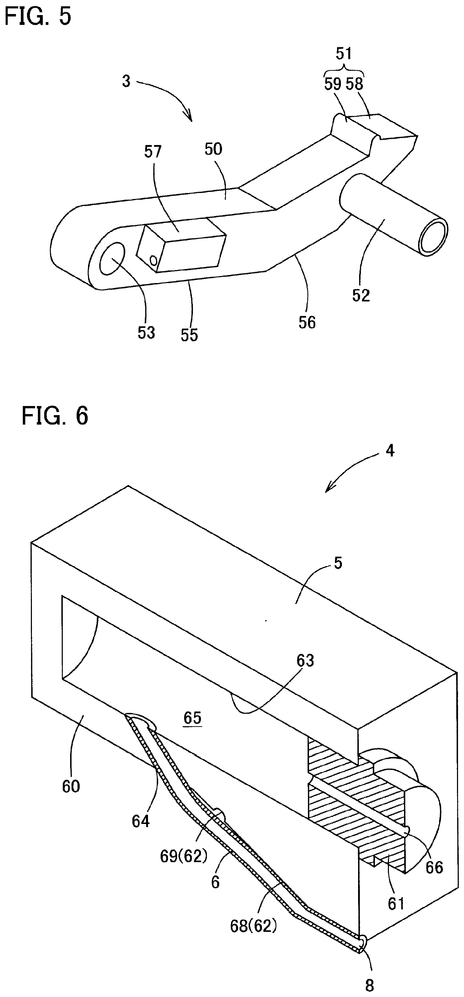

As illustrated in Fig. 5, the striking member 3 includes a main body portion 50, a striking portion 51, an operation portion 52, and a shaft hole 53.

The main body portion 50 is a hammer piece including a first extending portion 55, and a second extending portion 56 extending obliquely from an end portion of the first extending portion 55.

The first extending portion 55 includes a fixing portion 57 capable of fixing the end portion of the energizing member 7.

The striking portion 51 is a part that strikes a part of the tube member 6 with an impact force to discharge the liquid medicine 100 from the discharge port 8. That is, the striking portion 51 is a part that partially presses the tube member 6 to forcibly discharge the liquid medicine 100 in the tube member 6 from the discharge port 8.

As illustrated in Fig. 5, the striking portion 51 includes a flat portion 58, and a projecting portion 59 that projects with respect to the flat portion 58.

The projecting portion 59 is a ridge which forms a pair with a recessed portion 69 of the liquid medicine storage portion 5 and extends to cross the tube member 6 when assembled. That is, the projecting portion 59 extends in a direction intersecting an extension direction of the tube member 6, and is provided on the upstream side of the striking portion 51 in a capillarity flow direction of the liquid medicine 100 of the tube member 6.

The operation portion 52 is a part for operating the striking portion 51, and is a rod-shaped part that is provided on the second extending portion 56 and erected with respect to the second extending portion 56.

The shaft hole 53 is an insertion hole into which the shaft portion 25 can be inserted, and is a through-hole provided on the proximal end side of the first extending portion 55 and penetrating the first extending portion 55 in the thickness direction.

As illustrated in Fig. 6, the liquid medicine storage portion 5 is a container having a storage space 65 capable of storing the liquid medicine 100.

As illustrated in Fig. 7, the liquid medicine storage portion 5 includes a main body portion 60, and a cap portion 61.

As illustrated in Figs. 6 and 7, the main body portion 60 includes a struck portion 62, a storage hole 63, and a communication hole 64.

The struck portion 62 is a part which is struck with an impact force by the striking portion 51 of the striking member 3 via the tube member 6, and includes a flat portion 68, and the recessed portion 69 recessed with respect to the flat portion 68.

The recessed portion 69 is a recessed groove extending to cross the tube member 6 when assembled, and extends in a direction intersecting the extension direction of the tube member 6.

The storage hole 63 is a bottomed hole having a depth linearly in a front-rear direction from the front end surface of the liquid medicine storage portion 5, and is a hole that causes the storage space 65 to communicate with the outside.

The communication hole 64 is a through-hole that causes the storage space 65 to communicate with the outside and extends vertically or obliquely from the outside toward the storage space 65, and is also an insertion hole into which the tube member 6 can be inserted.

As illustrated in Fig. 7, the cap portion 61 is a closing portion that closes the storage hole 63, and has a through-hole 66.

The through-hole 66 is an air hole for outside air introduction, which introduces air into the storage space 65 from the outside, and is a through-hole penetrating in the thickness direction. That is, the through-hole 66 is a hole for introducing the outside air into the storage space 65 and constantly keeping the internal pressure and the external pressure in the liquid medicine storage portion 5 equal.

Note that the perimeter of the through-hole 66 may be subjected to hydrophilic processing or hydrophobic processing as necessary so that the liquid medicine 100 does not leak from the through-hole 66. In this manner, the liquid medicine 100 is less likely to leak from the through-hole 66.

As illustrated in Fig. 6, the tube member 6 is a member of which one end portion is inserted into the liquid medicine storage portion 5 and the other end portion constitutes the discharge port 8, and is a liquid delivery tube that delivers the liquid medicine 100 from the liquid medicine storage portion 5 to the discharge port 8.

The tube member 6 has a cylindrical body that is hollow inside and extends with a length. The tube member 6 of the present embodiment is a cylindrical tube, and has a circular cross-sectional shape.

The tube member 6 has an elastic region in which at least a part in the longitudinal direction is formed of an elastic body, and the elastic region is elastically deformable in a direction intersecting the longitudinal direction.

In the tube member 6 of the present embodiment, the entire region in the longitudinal direction is an elastic region.

The tube member 6 is not particularly limited as long as it is elastically deformable, and can be formed of, for example, at least one elastic material selected from the group consisting of polyethylene, polypropylene, silicone, fluororubber, polytetrafluoroethylene, nylon, polyethylene terephthalate, polybutylene terephthalate, polyethylene naphthalate, natural rubber, and synthetic rubber.

By using these elastic materials, a desired amount of the liquid medicine 100 can be reproducibly discharged.

In addition, the tube member 6 preferably has lipophilicity in a case where the liquid medicine is an oily preparation, and hydrophilicity in a case where the liquid medicine is an aqueous preparation, from the viewpoint of replenishing the liquid medicine 100 therein by capillarity.

The inner diameter (maximum inner diameter) of the tube member 6, that is, the smallest enclosing circle diameter of the internal space of the tube member 6 in the cross section orthogonal to the extension direction of the tube member 6 may be within a range in which the liquid medicine 100 can be transferred by capillarity, and is preferably 0.1 mm or more and 2 mm or less, and more preferably 0.3 mm or more and 1.5 mm or less.

The outer diameter (maximum outer diameter) of the tube member 6, that is, the smallest enclosing circle diameter of the outer surface of the tube member 6 in the cross section orthogonal to the extension direction of the tube member 6 is larger than the inner diameter of the tube member 6, and is preferably 0.2 mm or more and 5 mm or less.

The thickness (difference between the outer diameter and the inner diameter) of the tube member 6 is preferably 0.05 mm or more and 3 mm or less.

Within these ranges, the pressing force by the striking portion 51 is not excessively absorbed in the tube member 6, and can be efficiently transmitted to the internal liquid medicine 100.

The inner surface of the tube member 6 in liquid contact may be subjected to a hydrophobizing treatment or a hydrophilizing treatment as necessary.

The energizing member 7 is a member that energizes the striking portion 51 toward the liquid medicine storage portion 5.

Specifically, as illustrated in Fig. 3, the energizing member 7 is a torsion coil spring that includes a coil portion 80 and arm portions 81 and 82 extending from the coil portion 80, and receives a torsional moment around the central axis of the coil portion 80.

The coil portion 80 is an annular part, and the shaft portion 25 can be inserted at the center thereof.

The arm portions 81 and 82 are rod-shaped parts extending from the coil portion 80.

The discharge port 8 is an opening through which the liquid medicine 100 is discharged, and the discharge port 8 of the present embodiment is formed of an end portion of the tube member 6.

Subsequently, the positional relationship of the members of the dosing device 1 according to the first embodiment will be described.

As illustrated in Fig. 3, in the dosing device 1, the striking member 3, the liquid medicine storage portion 5, and the energizing member 7 are disposed in the case member 2. The first case portion 10 and the second case portion 11 are fixed to each other while facing each other such that the main body wall portions 20 and 30 face outward.

As illustrated in Fig. 2, in the dosing device 1, the side wall portions 21 to 24 of the first case portion 10 and the side wall portions 31 to 34 of the second case portion 11 constitute side surface portions 41 to 44 of the case member 2.

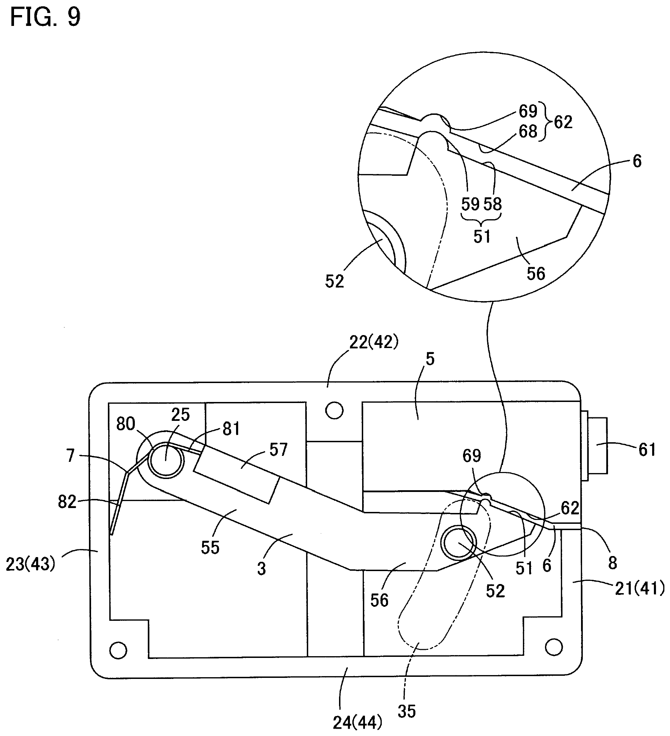

As illustrated in Fig. 3, the shaft portion 25 of the first case portion 10 is inserted through the shaft hole 53 of the striking member 3 and the coil portion 80 of the energizing member 7, and is inserted into and fixed to the shaft fixing hole 36. In the energizing member 7, the arm portion 81 is inserted into and fixed to the fixing portion 57, and the arm portion 82 is in contact with the side surface portion 43 of the case member 2. That is, as illustrated in Fig. 9, the energizing member 7 energizes the striking member 3 in the circumferential direction around the shaft portion 25 such that the striking portion 51 presses the struck portion 62 of the liquid medicine storage portion 5.

As illustrated in Fig. 2, the operation portion 52 is inserted through the guide groove 35, and a part thereof is exposed to the outside of the case member 2. That is, as illustrated in Fig. 9, the movable range of the operation portion 52 is restricted by the guide groove 35, and only movement in the circumferential direction around the shaft portion 25 is allowed.

The striking portion 51 receives the energizing force of the energizing member 7, and presses a part of the tube member 6 toward the liquid medicine storage portion 5.

As illustrated in Fig. 9, the tube member 6 extends downward (obliquely downward in the present embodiment) from the liquid medicine storage portion 5, and is partially bent and extends toward the side surface portion 41.

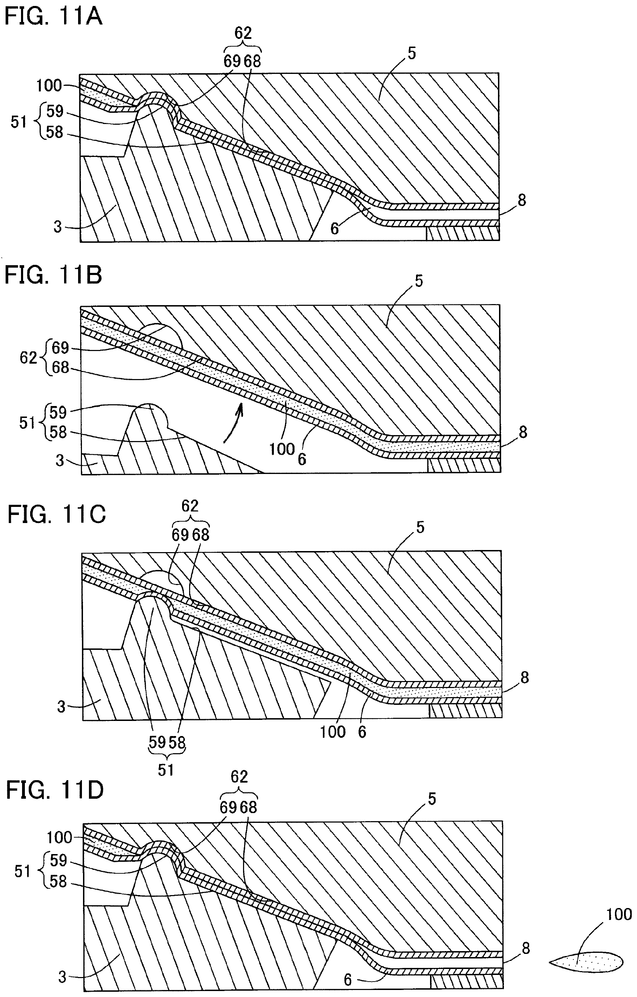

The tube member 6 is sandwiched between the struck portion 62 of the liquid medicine storage portion 5 and the striking portion 51 of the striking member 3, and a pressed portion 85 (Fig. 8) is elastically deformed into a flat shape by receiving the pressing force from the striking portion 51 toward the struck portion 62. That is, as illustrated in Fig. 11A, the inside of the tube member 6 is always elastically deformed by the pressing force of the striking portion 51, the flow path inside the pressed portion 85 is closed, and the flow of the liquid medicine 100 toward the discharge port 8 is blocked.

The projecting portion 59 of the striking portion 51 presses a portion of the tube member 6 corresponding to the recessed portion 69 of the struck portion 62.

The length of the striking part of the striking portion 51 illustrated in Fig. 8, that is, the length D in the longitudinal direction of the pressed portion 85 of the tube member 6 can be appropriately changed depending on the amount of the liquid medicine 100 to be discharged, but is preferably 1 mm or more and 30 mm or less, and more preferably 3 mm or more and 10 mm or less.

Within this range, the liquid medicine 100 in the tube member 6 can be discharged with good reproducibility.

Subsequently, a general process assumed in a case where a patient uses the dosing device 1 to administer the drug to the surface of the eyeball 101 will be described. Note that the dosing device 1 can take any posture, and can administer the liquid medicine 100 from any direction such as upward, downward, or sideways, but in order to facilitate understanding of the features of the present invention, a case where the liquid medicine 100 can be discharged in the horizontal direction will be described.

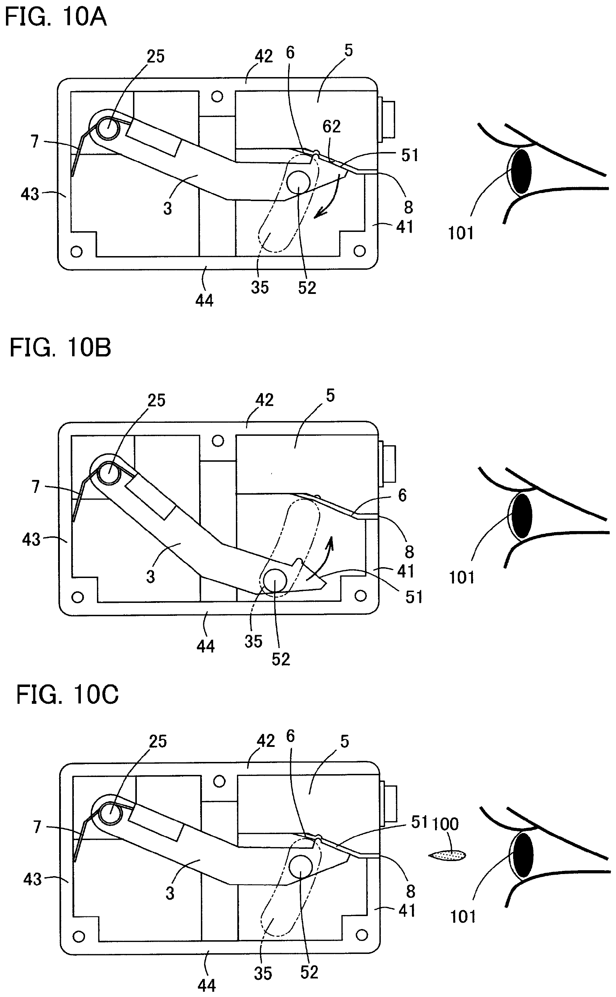

First, as illustrated in Figs. 10A and 10B, in a case where the operation portion 52 is operated in a state where the discharge port 8 faces the eyeball 101 and faces the horizontal direction, the striking member 3 rotates about the shaft portion 25 against the energizing force received from the energizing member 7, and the striking portion 51 is separated from the tube member 6.

In this case, as illustrated in Figs. 11A and 11B, the shape of the tube member 6 is restored to a cylindrical shape, and the liquid medicine 100 is automatically replenished from the liquid medicine storage portion 5 into the tube member 6 by capillarity.

Then, as illustrated in Figs. 10B and 10C, in a case where the user releases the operation portion 52, the striking member 3 rotates about the shaft portion 25 by the energizing force of the energizing member 7, and strikes the tube member 6. As a result, as illustrated in Figs. 11B to 11D, the tube member 6 is elastically deformed, and the liquid medicine 100 in the tube member 6 is pushed out to be discharged from the discharge port 8.

In this case, the liquid medicine 100 discharged from the discharge port 8 is a small amount of the liquid medicine 100, and is substantially linearly discharged from the discharge port 8 at least in a range of more than 0 cm and 0.5 cm or less.

Further, the discharge amount of the liquid medicine 100 from the discharge port 8 in this case is preferably 0.01 µL or more and 100 µL or less, more preferably 0.1 µL or more and 10 µL or less, and particularly preferably 0.1 µL or more and 7 µL or less.

Within this range, it is possible to prevent excessive administration of the liquid medicine 100, and it is possible to reduce problems caused by excessive administration, side effects caused by preservatives and the like.

A method of adjusting the discharge amount of the liquid medicine 100 from the discharge port 8 is not particularly limited, but the discharge amount can be adjusted by, for example, changing the length D of the striking part of the striking portion 51 or changing the inner diameter of the tube member 6.

Subsequently, the liquid medicine 100 that can be suitably used in the dosing device 1 will be described.

The liquid medicine 100 is not particularly limited as long as it is used as an ophthalmic drug.

The liquid medicine 100 is preferably any one of an oily preparation, an aqueous preparation, an aqueous suspension preparation, an oily suspension preparation, and an emulsion preparation, and more preferably an oily preparation.

The solvent that can be used as the base of the liquid medicine 100 is not particularly limited as long as it is liquid in a temperature range from a cold place to room temperature. Examples of the solvent include water, ethyl alcohol, ethylene glycol, polyethylene glycols, propylene glycol, medium-chain fatty acid triglycerides, glycerin, liquid paraffin, vegetable oil, animal oil, mineral oil, perfluorocarbons, and the like, and mixtures thereof.

Examples of the drug of the liquid medicine 100 include anti-inflammatory agents such as dipotassium glycyrrhizinate, epsilon aminocaproic acid, allantoin, berberine chloride, and berberine sulfate; antihistamines such as diphenhydramine hydrochloride and chlorpheniramine maleate; corneal therapeutic agents such as flavin adenine dinucleotide sodium, cyanocobalamin, retinols, pyridoxine hydrochloride, panthenol, sodium pantothenate, and sodium chondroitin sulfate; decongestants such as epinephrine, ephedrine hydrochloride, tetrahydrozoline hydrochloride, naphazoline hydrochloride, phenylephrine hydrochloride, and dl-methylephedrine hydrochloride; antibacterial agents such as ofloxacin, levofloxacin, tosufloxacin, gatifloxacin, gentamicin, sulfamethoxazole, and sulfisoxazole; β-lactam antibiotics such as cephalexin, imipenem, and meropenem; antifungal agents such as amphotericin B, ketoconazole, and fluconazole; cooling agents such as menthol, borneol, and camphor; therapeutic agents for glaucoma and ocular hypertension such as timolol maleate, pilocarpine hydrochloride, carteolol, latanoprost, tafluprost, travoprost, and Ripasudil; therapeutic agents for cataract such as phacolysine, glutathione, pirenoxine, and sodium pentacesylsulfonate; antibiotics such as chloramphenicol, colistin, and erythromycin; immunosuppressants such as rapamycin, tacrolimus, and cyclosporin; adrenocortical hormone agents such as hydrocortisone acetate, prednisolone acetate, dexamethasone, triamcinolone acetonide, and fluorometholone; carbonic anhydrase inhibitors such as brinzolamide, dorzolamide, and methazolamide; α2 agonists such as brimonidine; α1 blockers such as bunazosin; antioxidants such as tocopherols and vitamin C; mucosal repair agents (dry eye therapeutic agents) such as hyaluronic acid, chondroitin sulfate ester, gefarnate, vitamin U, and rebamipide; local anesthetics such as lidocaine hydrochloride and dibucaine hydrochloride; test agents such as fluorescein, rose bengal, cyclopentolate, and tropicamide; protein preparations; antibody preparations; and nucleic acid preparations.

The viscosity of the liquid medicine 100 is not particularly limited, but is preferably 10,000 cps or less, more preferably 1,000 cps or less, and still more preferably 300 cps or less.

With the dosing device 1 of the first embodiment, since the liquid medicine 100 is discharged from the discharge port 8 by operating the operation portion 52, the patient does not need to face upward, and the liquid medicine 100 can be administered from any direction without depending on gravity. Therefore, even a person who is difficult to take medication such as a patient who is difficult to change his/her posture, an elderly person, a rheumatism patient, or a patient with poor grip strength can accurately administer a predetermined small amount of the liquid medicine 100 to the surface of the eyeball 101 of the patient. As a result, it is possible to improve patient convenience, prevent excessive administration of the liquid medicine 100, and reduce side effects of the preservatives.

Further, with the dosing device 1 of the first embodiment, since the patient does not need to face upward, in a case where the test agent is instilled into the eye in the ophthalmic examination room, the liquid medicine 100 can be administered while the patient is sitting, and convenience can be improved.

With the dosing device 1 of the first embodiment, the amount of the liquid medicine to be discharged (for example, 0.001 to 7 µL) is much smaller than the amount of drops (25 to 50 µL) by dropwise administration of conventional eye drops. Therefore, the amount of the liquid medicine 100 discharged onto the surface of the eyeball 101 is small, and there is no possibility that the liquid medicine 100 flows out of the eye, and all the liquid medicine can be effectively used.

In addition, with the dosing device 1 of the first embodiment, it is possible to administer a smaller amount of the liquid medicine 100 than that by the dropwise administration from the conventional eye-drop container, and the influence of the liquid medicine 100 on the living body is also reduced. Therefore, the degree of freedom in design of the liquid medicine 100 is high, for example, so that it is possible to provide a wide pH range, a wide osmotic pressure range, and a wide viscosity range in liquid medicine design. In addition, the oily liquid medicine 100 can also be administered without disquiet.

Furthermore, with the dosing device 1 of the first embodiment, since the liquid medicine 100 to be administered to the surface of the eyeball 101 can be reduced to a small amount of droplets, it is possible to prevent the patient from feeling disquiet or feeling irritation in the eyeball 101.

With the dosing device 1 of the first embodiment, by operating the operation portion 52, a part of the tube member 6 is struck by the striking portion 51, and a part of the liquid medicine 100 in the tube member 6 is pushed out and discharged from the discharge port 8. Therefore, a small amount of the liquid medicine 100 can be linearly discharged.

With the dosing device 1 of the first embodiment, the tube member 6 delivers liquid from the liquid medicine storage portion 5 toward the discharge port 8 by capillarity. Therefore, the liquid medicine 100 can be automatically and easily replenished into the tube member 6.

With the dosing device 1 of the first embodiment, since the entire region of the tube member 6 constitutes the elastic region, the tube member 6 is not needed to combine a plurality of types of elastic materials, leading to easy manufacturing.

With the dosing device 1 of the first embodiment, by operating the operation portion 52, the posture of the striking portion 51 is changed against the energizing force from the energizing member 7, and the striking portion 51 strikes the tube member 6 with the energizing force. That is, since the striking can be made by the energizing force of the energizing member 7, the liquid medicine 100 can be more accurately discharged from the discharge port 8.

With the dosing device 1 of the first embodiment, by operating the operation portion 52 in a state where the discharge port 8 faces the horizontal direction, the liquid medicine 100 is substantially linearly discharged from the discharge port 8 at least in a range of more than 0 cm and 0.5 cm or less. Therefore, even in a case where a third person such as a doctor, a nurse, or a caregiver performs medication, the medication can be easily performed.

With the dosing device 1 of the first embodiment, the projecting portion 59 is provided on the upstream side of the striking portion 51 in the flow direction of the liquid medicine 100 of the tube member 6 due to the capillarity. Therefore, in a case where the tube member 6 is struck by the striking portion 51, the projecting portion 59 can block the movement of the liquid medicine 100 toward the liquid medicine storage portion 5, and the liquid medicine 100 can be more accurately discharged from the discharge port 8.

Subsequently, a dosing device 500 of a second embodiment of the present invention will be described. The same components as those of the dosing device 1 of the first embodiment are denoted by the same reference numerals, and the description thereof will be omitted. The same applies hereinafter. Similarly to the first embodiment, the dosing device 500 can take any posture, and can administer the liquid medicine 100 from any direction such as upward, downward, or sideways, but in order to facilitate understanding of the features of the present invention, a description will be given on the basis of a posture in which the liquid medicine 100 can be discharged in the horizontal direction and an operation member 512 is on the upper side.

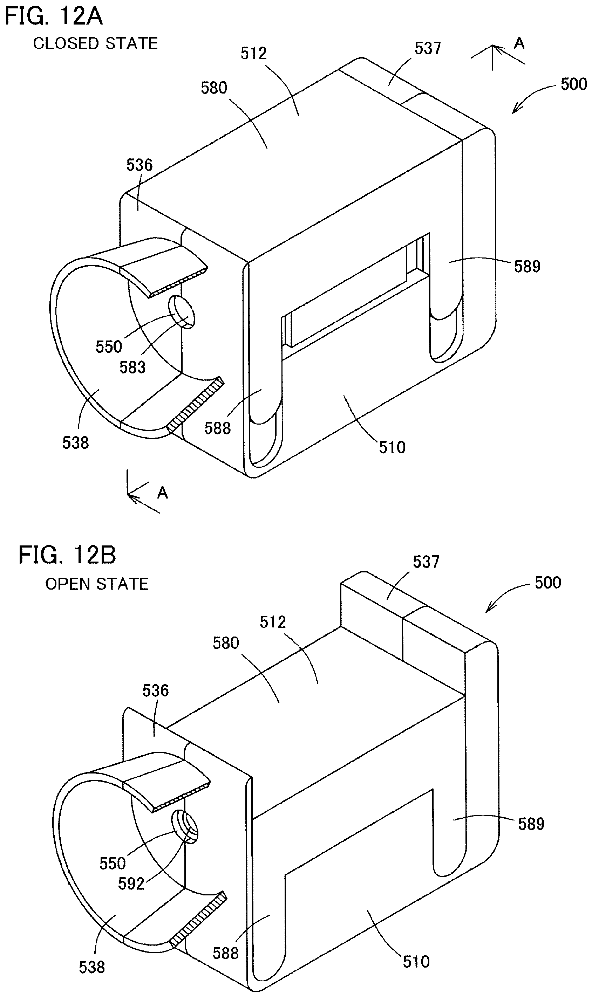

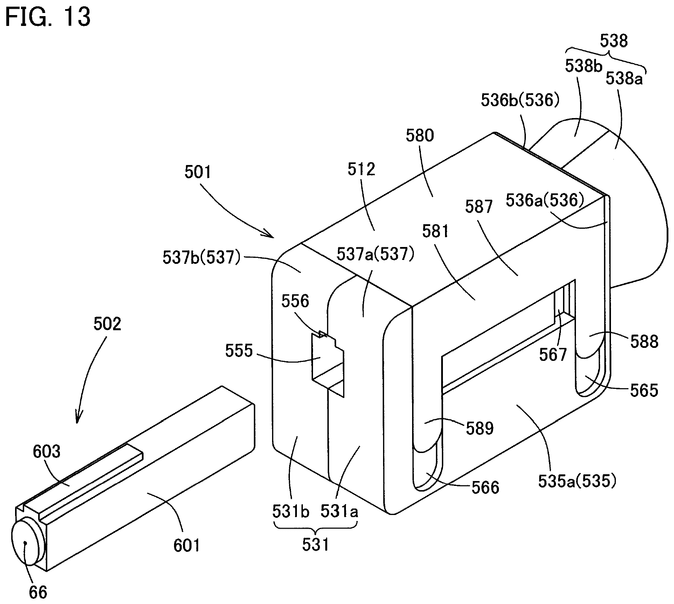

As illustrated in Figs. 12 and 13, the dosing device 500 of the second embodiment includes a main body portion 501, and a liquid medicine cartridge 502 (liquid cartridge), and the liquid medicine cartridge 502 is attachable to and detachable from the main body portion 501.

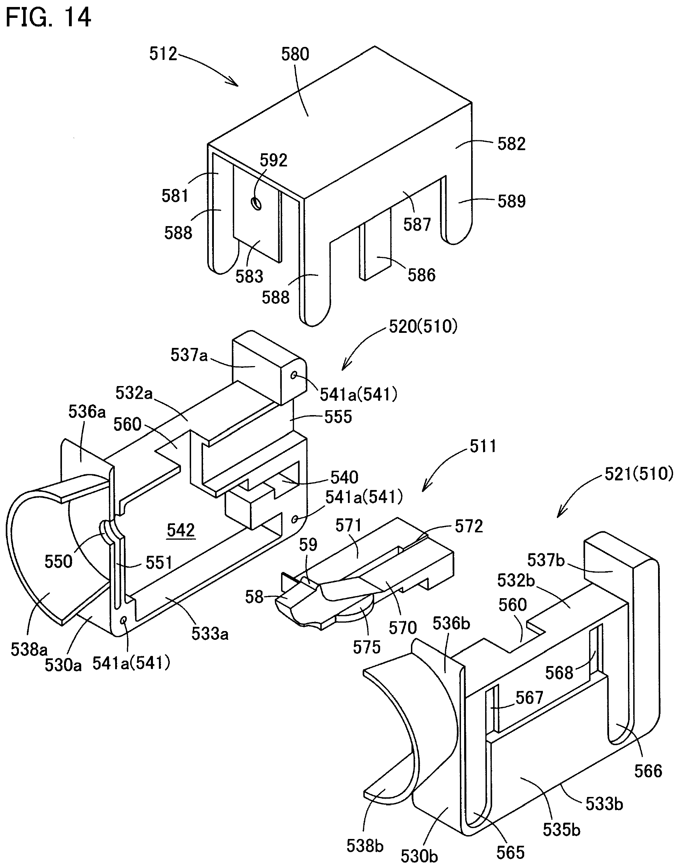

As illustrated in Fig. 14, the main body portion 501 includes a case member 510, a striking member 511, and the operation member 512.

As illustrated in Fig. 14, the case member 510 includes a pair of case portions 520 and 521.

As can be seen from Figs. 13, 14, and 16, the case member 510 includes a front wall portion 530 (530a, 530b), a back wall portion 531 (531a, 531b), a top wall portion 532 (532a, 532b), a bottom wall portion 533 (533a, 533b), a side wall portion 535 (535a, 535b), a front side guide wall portion 536 (536a, 536b), a back side guide wall portion 537 (537a, 537b), a position fixing portion 538 (538a, 538b), a fixing point for striking 540, and a fitting portion 541 (541a, 541b).

The case member 510 includes an internal space 542 surrounded by the wall portions 530 to 533 and 535.

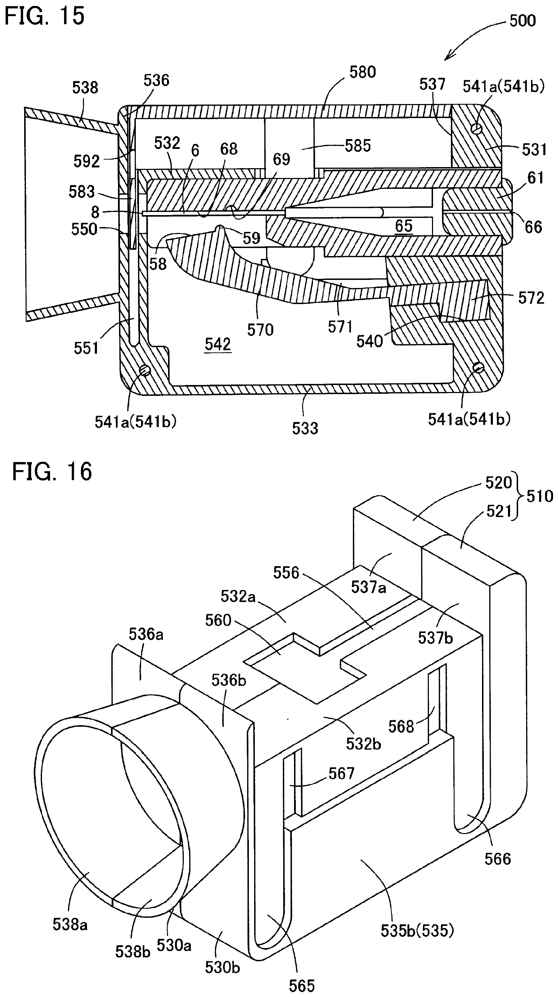

As illustrated in Figs. 14 and 15, the front wall portion 530 includes a liquid medicine passage hole 550 (liquid passage hole) penetrating in the front-rear direction from the outside toward the internal space 542, and a case-side restriction groove 551 extending in the vertical direction.

The liquid medicine passage hole 550 is a hole through which the liquid medicine discharged from the discharge port 8 of the liquid medicine cartridge 502 passes.

The case-side restriction groove 551 is a groove that restricts the movement direction of a closing portion 583 that closes the discharge port 8 of the liquid medicine cartridge 502 of the operation member 512, and the depth thereof is longer than the length of the closing portion 583 of the operation member 512.

As illustrated in Fig. 13, the back wall portion 531 includes an attachment portion 555 to which the liquid medicine cartridge 502 can be attached.

As illustrated in Fig. 14, the attachment portion 555 is an attachment hole extending from the outside toward the internal space 542, and is a communication hole continuous with the internal space 542.

As illustrated in Figs. 13 and 16, the attachment portion 555 includes a restriction groove 556 that restricts the direction of the liquid medicine cartridge 502.

The top wall portion 532 includes a communication hole 560 penetrating in the vertical direction from the outside toward the internal space 542 and communicating with the internal space 542.

The communication hole 560 is an insertion hole into which an operation-side pressing portion 585 and an operation-side engagement portion 586 of the operation member 512 can be inserted, and is also continuous with the restriction groove 556 of the attachment portion 555.

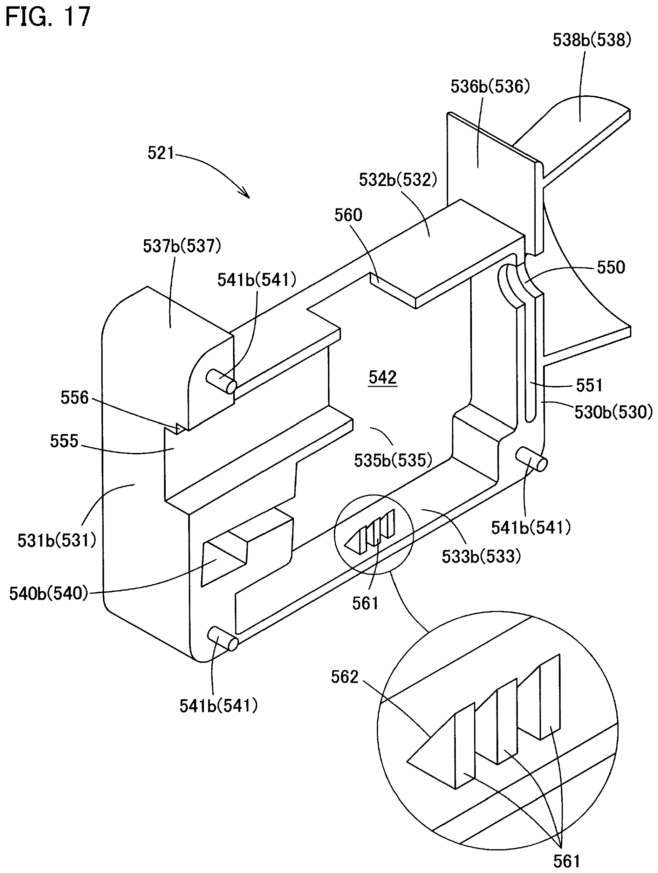

As illustrated in Fig. 17, the bottom wall portion 533b of one case portion 521 includes guide portions 561.

The guide portions 561 are triangular plate-shaped parts that guide a main body plate portion 595 of the operation member 512 toward the side wall portion 535b.

The guide portions 561 of the present embodiment are configured by arranging a plurality of right triangular plate-shaped pieces in the front-rear direction, and an inclined portion 562 is formed on the side of the guide portion 561 close to the side wall portion 535 by an inclined surface of each plate-shaped piece.

As illustrated in Fig. 14, the side wall portion 535 includes, on the outer surface, slide grooves 565 and 566 extending from the upper end portion toward the lower end portion in the vertical direction, and locking grooves 567 and 568.

The slide grooves 565 and 566 are restriction grooves that restrict the sliding direction of leg portions 588 and 589 of the operation member 512 in the vertical direction.

The locking grooves 567 and 568 are parts that extend in the vertical direction, are locked with locking claws 590 and 591 of side wall portions 581 and 582, and prevent separation of the operation member 512 from the case member 510.

That is, in the locking grooves 567 and 568, the inner wall portions positioned at the upper end portions of the locking grooves 567 and 568 constitute locking surfaces for locking the locking claws 590 and 591 of the side wall portions 581 and 582.

The guide wall portions 536 and 537 are parts that guide the movement direction of the operation member 512 in the vertical direction, and are wall portions projecting upward from the top wall portion 532.

The position fixing portion 538 is a part that fixes a distance and a position between discharge port 8 and the eyeball 101 of the user.

The fixing point for striking 540 is a part that holds a connection portion 572 of the striking member 511.

The fitting portions 541 (541a, 541b) are parts that are fitted to each other and fix the case portions 520 and 521.

In the case portions 520 and 521, the fitting portion 541a of one case portion 520 is a recessed portion as illustrated in Fig. 14, and the fitting portion 541b of the other case portion 521 is a projecting portion as illustrated in Fig. 17.

As illustrated in Fig. 14, the striking member 511 includes a striking portion 570, an elastic portion 571, and the connection portion 572.

The striking portion 570 includes the flat portion 58, the projecting portion 59 projecting with respect to the flat portion 58, and an engagement portion 575.

The engagement portion 575 is a part that engages with a locking surface 597 of the operation member 512 in a state with no load on the operation member 512.

The elastic portion 571 is an elastically deformable member, specifically, a leaf spring.

The elastic portion 571 is supported in a cantilever manner by the case member 510 via the connection portion 572, and extends from the connection portion 572 toward the same direction (forward direction) as the striking portion 570.

The connection portion 572 is a part that is fixed to the fixing point for striking 540 and is connected to the case member 510, and is also a part that connects the proximal end portion of the striking portion 570 and the proximal end portion of the elastic portion 571.

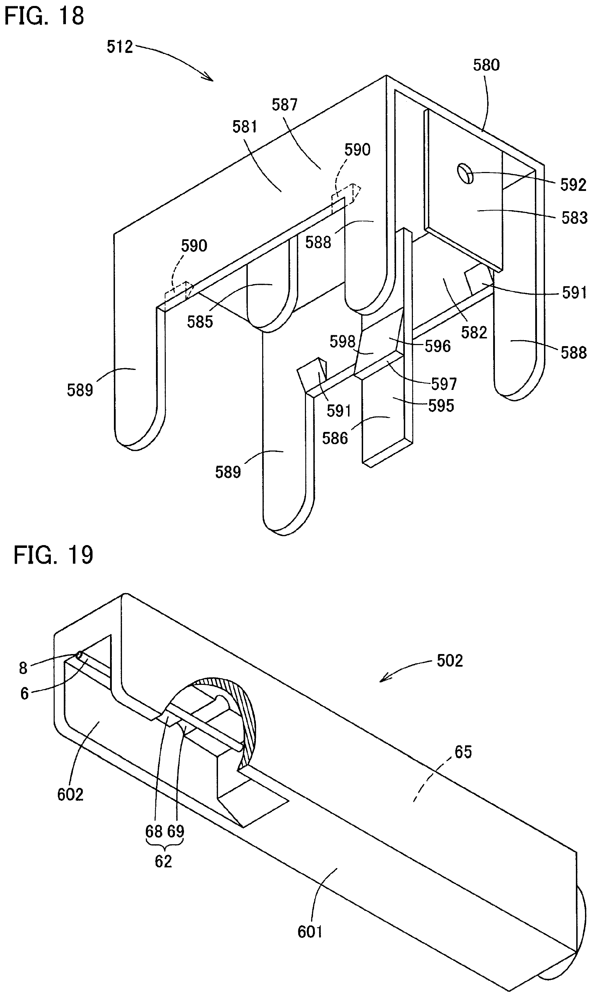

As illustrated in Figs. 14 and 18, the operation member 512 includes an operation surface portion 580, the side wall portions 581 and 582, the closing portion 583, the operation-side pressing portion 585, and the operation-side engagement portion 586.

The operation surface portion 580 is a part pressed to be operated by the user, and is a plate-shaped part constituting the top surface of the dosing device 500.

As illustrated in Fig. 18, the side wall portions 581 and 582 are wall portions hanging downward from the operation surface portion 580, and include a main body portion 587 and the leg portions 588 and 589.

The main body portion 587 is a plate-shaped part extending in the front-rear direction, and includes the locking claws 590 and 591, which lock separation of the operation member 512 from the case member 510, at a lower end portion or in the vicinity of the lower end portion.

The leg portions 588 and 589 are projecting portions that project downward from the lower end portion of the main body portion 587.

As illustrated in Fig. 18, the closing portion 583 is a part that closes the discharge port 8 of the liquid medicine cartridge 502.

The closing portion 583 has a plate-shaped body extending downward from the operation surface portion 580, and includes an operation-side through-hole 592 penetrating in the thickness direction.

The operation-side through-hole 592 is an open hole that opens the discharge port 8 of the liquid medicine cartridge 502 in a case where the operation member 512 is operated.

The operation-side pressing portion 585 is a projection portion extending downward from the operation surface portion 580, and is a part that presses the elastic portion 571 of the striking member 511 at an end portion in the extension direction.

As illustrated in Fig. 18, the operation-side engagement portion 586 includes the main body plate portion 595 and a locking portion 596.

The main body plate portion 595 is a projection portion extending downward from the operation surface portion 580, and is a part that presses the guide portions 561 of the case portion 521 at an end portion in the extension direction.

The locking portion 596 is a part that is provided at an intermediate portion of the main body plate portion 595 in the extension direction, engages with the engagement portion 575 of the striking portion 570 of the striking member 511, and locks the upward movement of the striking member 511.

The locking portion 596 is a projecting portion that projects in a direction intersecting the extension direction of the main body plate portion 595, and includes the locking surface 597, and an inclined surface 598.

The locking surface 597 is a surface that faces downward and abuts on and locks the engagement portion 575 of the striking portion 570, and is a vertical surface standing perpendicularly to the inner surface of the main body plate portion 595.

The inclined surface 598 is a surface inclined at an acute angle with respect to the inner surface of the main body plate portion 595.

As illustrated in Fig. 19, the liquid medicine cartridge 502 includes a liquid medicine storage portion 601 and the tube member 6.

The liquid medicine storage portion 601 is a rectangular parallelepiped member that has the storage space 65 therein and extends in an insertion direction of the liquid medicine cartridge 502.

The liquid medicine storage portion 601 includes a cutout portion 602 from the distal end portion side of the insertion direction toward the base end portion side, and the tube member 6 is disposed in the cutout portion 602.

The struck portion 62 is provided on the inner surface of the cutout portion 602, and the flat portion 68 and the recessed portion 69 are formed.

As illustrated in Fig. 13, the liquid medicine storage portion 601 includes a ridge portion 603 extending from the distal end portion of the insertion direction toward the base end portion.

Next, the positional relationships of respective members of the dosing device 500 in a state where the operation member 512 is not operated, that is, in a no-load state will be described.

As illustrated in Fig. 15, in the dosing device 500, the striking member 511 and the liquid medicine cartridge 502 are disposed in the case member 510, and the operation member 512 covers the outside of the case member 510.

As illustrated in Fig. 14, the case member 510 is shaped such that the case portions 520 and 521 are connected so that the side wall portions 535a and 535b are on the outside.

The fitting portion 541b of the case portion 521 is inserted and fitted into the fitting portion 541a of the case portion 520.

As illustrated in Fig. 15, in the striking member 511, the connection portion 572 is fixed to the fixing point for striking 540 of the case member 510, and the striking portion 570 and the elastic portion 571 are supported in a cantilever manner by the case member 510. That is, the proximal end portions of the striking portion 570 and the elastic portion 571 are fixed to the fixing point for striking 540, and the distal end portions are free ends.

As illustrated in Figs. 12 and 13, in the operation member 512, the operation surface portion 580 is flush with the distal end surface of the guide wall portions 536 and 537 of the case member 510 in the projection direction.

The closing portion 583 of the operation member 512 is inserted into the case-side restriction groove 551 of the case member 510, and closes the liquid medicine passage hole 550. That is, the dosing device 500 is in a closed state in which the liquid medicine passage hole 550 is closed by the closing portion 583.

The liquid medicine cartridge 502 is attached to the attachment portion 555, and the ridge portion 603 is inserted into the restriction groove 556 of the case member 510.

Subsequently, a dosing method at the time of administration to a user using the dosing device 500 will be described.

First, as can be seen from Fig. 13, the liquid medicine cartridge 502 containing a desired liquid medicine is inserted into the attachment portion 555 of the main body portion 501.

In this case, the liquid medicine cartridge 502 is moved linearly along the restriction groove 556 of the attachment portion 555, and the tube member 6 is disposed to face the striking portion 570.

In this case, since the dosing device 500 is in the closed state as illustrated in Fig. 12A, the liquid medicine passage hole 550 is closed by the closing portion 583, and the discharge port 8 of the tube member 6 faces the closing portion 583.

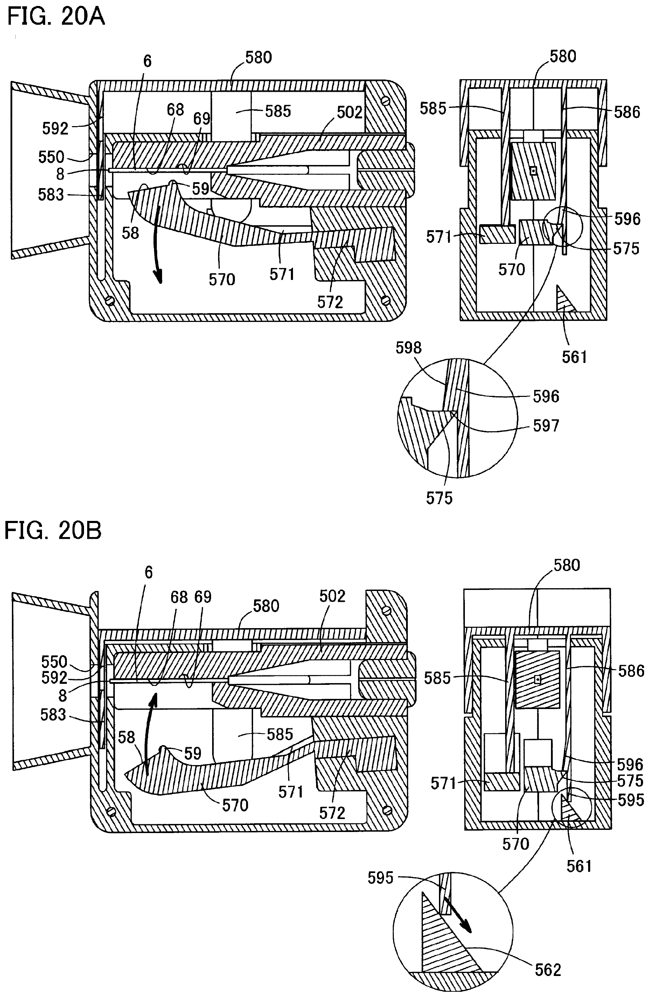

Subsequently, as illustrated in Figs. 20A and 20B, in a case where the user presses and operates the operation surface portion 580 of the operation member 512 with a finger, the operation-side pressing portion 585 of the operation member 512 presses the elastic portion 571, the locking surface 597 of the locking portion 596 of the operation-side engagement portion 586 presses the engagement portion 575 of the striking portion 570, and the elastic portion 571 and the striking portion 570 rotate about the connection portion 572.

In a case where the operation surface portion 580 of the operation member 512 is pressed to be operated, and the elastic portion 571 and the striking portion 570 further rotate in a state where the distal end surface of the main body plate portion 595 is in contact with the guide portions 561 of the case member 510 as illustrated in Fig. 20B, the main body plate portion 595 is moved outward along the inclined portion 562 of the guide portions 561, and the engagement between the locking surface 597 of the locking portion 596 and the engagement portion 575 of the striking portion 570 is released.

In this case, the operation-side through-hole 592 is linearly aligned with the liquid medicine passage hole 550, the liquid medicine passage hole 550 is opened, and the open posture illustrated in Fig. 12B is obtained.

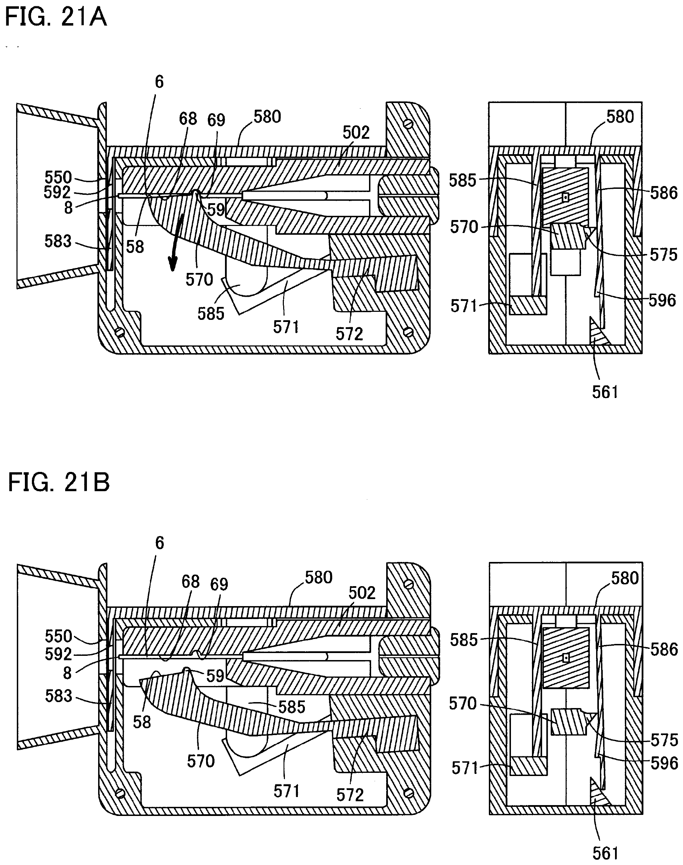

In a case where the engagement between the locking surface 597 of the locking portion 596 and the engagement portion 575 of the striking portion 570 is released, as illustrated in Fig. 21A, the striking portion 570 rotates about the connection portion 572 by its own restoring force, and the striking portion 570 strikes a part of the tube member 6 with an impact force.

Then, the liquid medicine 100 in the tube member 6 is linearly discharged from the discharge port 8 by the striking force, passes through the liquid medicine passage hole 550 and the operation-side through-hole 592, and reaches the eyeball 101 of the user.

In this case, a centrifugal force about the connection portion 572 acts on the striking portion 570, and the striking portion 570 strikes the elastic region of the tube member 6 by inertia exceeding the height of the connection portion 572.

As illustrated in Fig. 21A, after striking the tube member 6, the striking portion 570 tries to return to the position in the closed state again by the restoring force in the opposite direction.

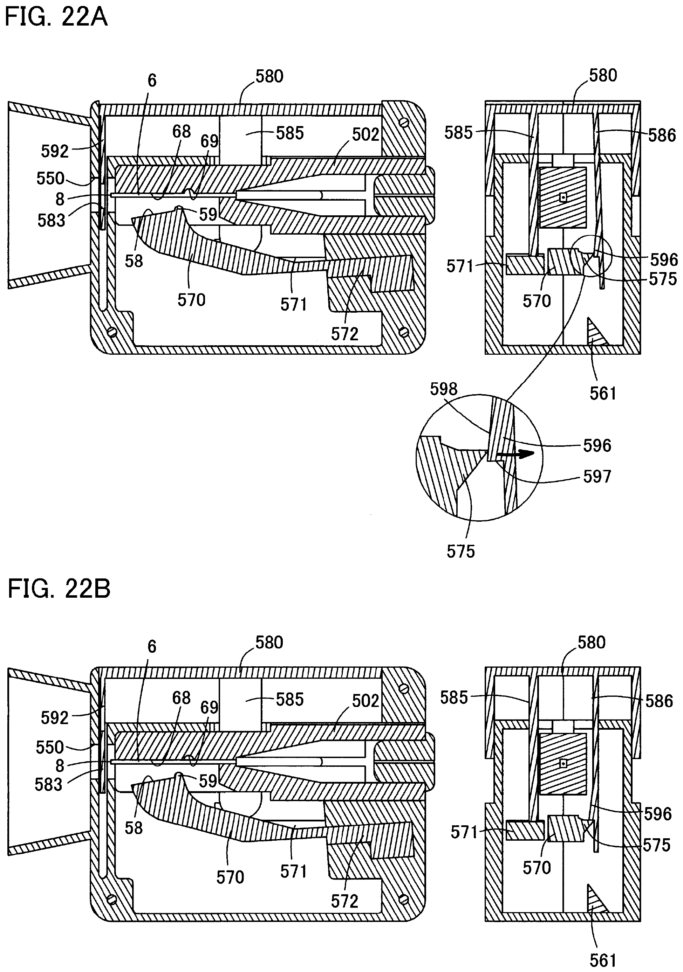

In a case where the user stops the pressing operation on the operation surface portion 580, as illustrated in Figs. 21 B and 22A, the operation member 512 is moved upward by the restoring force of the elastic portion 571, and as illustrated in Figs. 22A and 22B, the engagement portion 575 of the striking portion 570 is moved along the inclined surface 598, and the engagement portion 575 of the striking portion 570 engages with the locking surface 597 by climbing over the inclined surface 598. In addition, the closing portion 583 is moved along the case-side restriction groove 551 along with the upward movement of the operation member 512, and returns to the closed state in which the liquid medicine passage hole 550 illustrated in Fig. 12A is closed by the closing portion 583.

With the dosing device 500 of the present embodiment, since the liquid medicine cartridge 502 is attachable to and detachable from the main body portion 501, it is possible to easily replace the liquid medicine in a case where the liquid medicine runs out or in a case where it is desired to drop a different liquid medicine.

With the dosing device 500 of the present embodiment, in a case where the pressing operation is not performed, the dosing device 500 is in the closed state in which the closing portion 583 closes the liquid medicine passage hole 550, and in a case where the pressing operation is performed, the closing portion 583 is moved with the pressing operation to automatically open the liquid medicine passage hole 550. That is, in a case where the pressing operation is not performed, since the discharge port 8 of the tube member 6 is always protected by the closing portion 583, the discharge port 8 is not exposed to the outside except in a case where the liquid medicine cartridge 502 is replaced or in a case where eye drops are applied, and the discharge port 8 is less likely to be contaminated.

With the dosing device 500 of the present embodiment, the striking portion 570 is elastically deformed by the pressing operation, and the restoring force causes the striking portion 570 to strike at least a part of the elastic region at a position higher than the connection portion 572 with an impact force. That is, like a pendulum, since the striking portion 570 strikes the tube member 6 by the restoring force and the inertial force of the striking portion 570, it is not necessary to further provide a energizing member for striking with an impact force, and the cost can be reduced.

Subsequently, a dosing device 700 of a third embodiment of the present invention will be described.

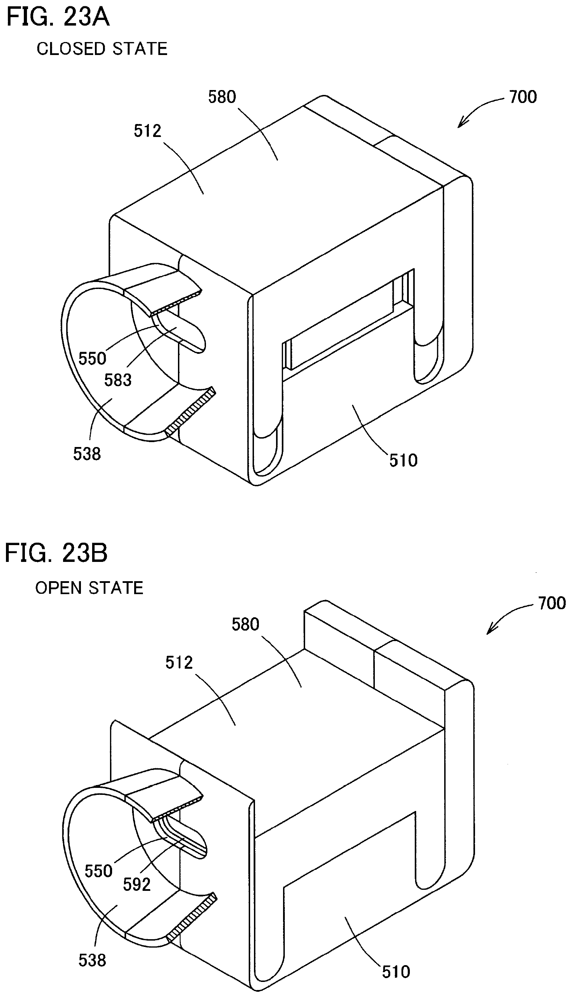

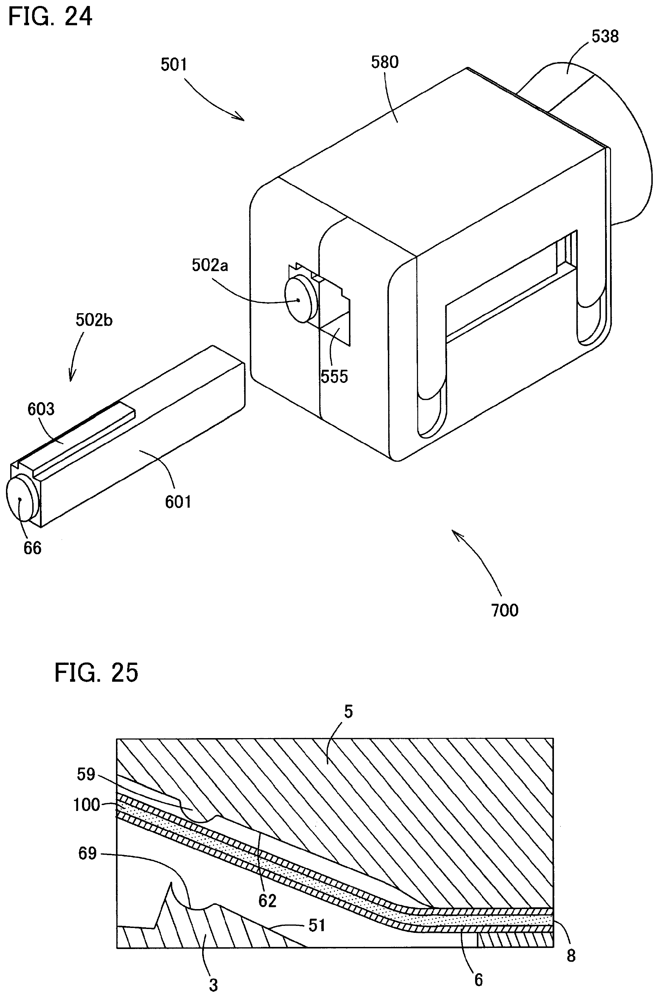

As illustrated in Figs. 23 and 24, in the dosing device 700 of the third embodiment, a plurality of liquid medicine cartridges 502a and 502b can be individually attached to and detached from the attachment portion 555 of the main body portion 501.

Similarly to the dosing device 500 of the second embodiment, in the dosing device 700, the discharge ports 8 and 8 of the liquid medicine cartridges 502a and 502b are blocked by the closing portion 583 in the closed state, the state is changed to the open state by the pressing operation on the operation surface portion 580, the discharge ports 8 and 8 of the liquid medicine cartridges 502a and 502b overlap the operation-side through-hole 592 to be opened, and the liquid medicine of the liquid medicine cartridges 502a and 502b can be simultaneously discharged from the discharge ports 8 and 8.

With the dosing device 700 of the third embodiment, since the plurality of liquid medicine cartridges 502a and 502b can be attached to the main body portion 501, different types of liquid medicines can be administered simultaneously, and the patient can be released from the troublesome multi-drug administration.

With the dosing device 700 of the third embodiment, since the liquid medicine cartridges 502a and 502b can be replaced individually, the liquid medicines in the liquid medicine cartridges 502a and 502b can be used to the end.

In the above-described embodiments, the tube member 6 has a uniform inner diameter from the liquid medicine storage portion 5 to the discharge port 8, but the present invention is not limited thereto. A narrowed portion may be provided in a part of the tube member 6 between the inside of the liquid medicine storage portion 5 and the discharge port 8. For example, an orifice may be provided at an end portion of the struck part of the tube member 6 on the discharge port 8 side. In this way, the discharge distance of the liquid medicine 100 can be improved.

In the above-described embodiment, the recessed portion 69 is provided in the struck portion 62, and the projecting portion 59 is provided in the striking portion 51, but the present invention is not limited thereto. As illustrated in Fig. 25, the projecting portion 59 may be provided on the struck portion 62, and the recessed portion 69 may be provided on the striking portion 51.

In addition, the projecting portion 59 and the recessed portion 69 may not be provided in the struck portion 62 and the striking portion 51. That is, the struck portion 62 and the striking portion 51 may be formed only of the flat portion 68 and the flat portion 58.

In the embodiments described above, the cross-sectional shape of the tube member 6 is a circular shape, but the present invention is not limited thereto. The cross-sectional shape of the tube member 6 may be a polygonal shape such as a triangle, a quadrangle, a pentagon, or a hexagon, or may be a flat shape, an elliptical shape, or an oval shape.

In the embodiments described above, the tube member 6 has a uniform wall thickness, but the present invention is not limited thereto. There may be a partially thin portion. For example, the struck part of the tube member 6 may be thicker than other portions.

In the embodiments described above, the entire tube member 6 is formed of an elastic material, so that the entire region of the tube member 6 is an elastic region, but the present invention is not limited thereto. At least a part of the tube member 6 in the longitudinal direction, for example, only the region where the tube member 6 is struck by the striking portion 51 or only the vicinity of the region where the tube member 6 is struck by the striking portion 51 may be the elastic region.

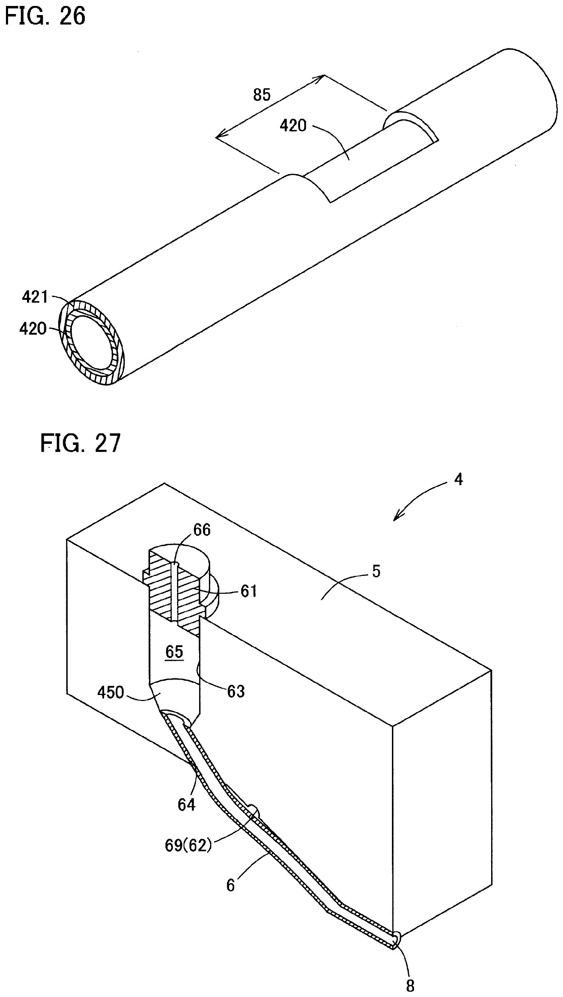

In the embodiments described above, the tube member 6 is formed of one elastic material, but the present invention is not limited thereto. As illustrated in Fig. 26, the tube member 6 may have a two-layer structure of an elastic layer 420 formed of an elastic material and a hard layer 421 formed of a hard material, or may have three or more layers. That is, the tube member 6 may have a multilayer structure. In this case, the elastic layer 420 is preferably exposed as an elastic region at least in the pressed portion 85 by the striking portion 51.

In the embodiments described above, the striking member 3 changes its posture against the energizing force from the energizing member 7 to cause the striking by the striking portion 51 using the reaction force, but the present invention is not limited thereto. The striking member 3 may be moved against the energizing force from the energizing member 7 to cause the striking by the striking portion 51 using the reaction force.

In the embodiments described above, the case where the dosing devices 1, 500, and 700 are used for eye drops has been described, but the present invention is not limited thereto. The dosing devices 1, 500, and 700 may be used for nasal, ear, oral, or transdermal administration.

In the embodiments described above, the storage space 65 of the liquid medicine storage portion 5 extends in the horizontal direction in a posture in which the discharge port 8 of the dosing device 1 faces the horizontal direction, but the present invention is not limited thereto. For example, as illustrated in Fig. 27, the storage space 65 of the liquid medicine storage portion 5 may extend in the vertical direction while the discharge port 8 of the dosing device 1 faces the horizontal direction. In this case, it is preferable that a part or the whole of the bottom portion of the liquid medicine storage portion 5 includes an introduction portion 450 that is inclined downward toward the tube member 6 and guides the liquid medicine 100 to the tube member 6.

In the first embodiment described above, the liquid medicine unit 4 is not attachable to and detachable from the case member 2, but the present invention is not limited thereto. The liquid medicine unit 4 may be attachable to and detachable from the case member 2. In this way, the used liquid medicine unit 4 can be replaced with a new liquid medicine unit 4 as in the cartridge system. Therefore, other members other than the liquid medicine unit 4 can be reused.

The dosing device 1 according to the embodiment described above may be provided with a protective cover for protecting the discharge port 8 and the tube member 6 as necessary.

The dosing device 1 according to the first embodiment described above may be provided with a fixing jig or the like for fixing the distance or position between the discharge port 8 and the eyeball 101 as necessary.

In the embodiments described above, the tube member 6 is struck by changing the posture of the striking portions 51 and 570, but the present invention is not limited thereto. The striking portions 51 and 570 may be moved relative to the tube member 6 to strike the tube member 6.

In the second embodiment described above, the closed state in which the closing portion 583 closes the liquid medicine passage hole 550 and the open state in which the closing portion 583 is moved relative to the case member 510 to open the liquid medicine passage hole 550 are changed by operating the operation member 512, but the present invention is not limited thereto. The closed state may be changed to the open state by changing the posture of the closing portion 583 with respect to the case member 510 by operating the operation member 512.

In the embodiments described above, the case where the liquid discharge device is used as the dosing device has been described, but the present invention is not limited thereto. The liquid discharge device of the present invention can be used in other applications. For example, it can also be used as a liquid dispensing pipette, an adhesive applicator, a dropper for aromatic oil, perfume, or the like, an oil filling device for precision parts, an animal medicine administration device, or an insecticide discharge device.

In the embodiments described above, each component can be freely replaced or added between the embodiments as long as it is included in the technical scope of the present invention.

### [Examples]

Hereinafter, the present invention will be specifically described with reference to examples. Note that the present invention is not limited to the following examples, and can be appropriately modified without changing the gist thereof.

### (Example 1)

In the dosing device according to the first embodiment described above, a silicone tube having an inner diameter of 0.5 mm and an outer diameter of 0.6 mm was used as the tube member, and the length of the striking part of the striking portion (the length D of the pressed portion of the tube member) was set to 6 mm. In addition, medium-chain fatty acid triglyceride (specific gravity: 0.953 g/cm³) was used as the liquid medicine. This was designated as Example 1.

### (Example 2)

The same procedure as in Example 1 was carried out except that an orifice having an orifice diameter of 0.15 mm was provided at the discharge port of the tube member, and this procedure was designated as Example 2.

### (Discharge amount test)

The liquid medicine is transferred from the liquid medicine reservoir to the tube member by capillarity, and the influence of the discharge interval (administration interval: 1 second, 5 seconds, 10 seconds, 20 seconds) on the discharge amount was examined.

In this case, a medium-chain fatty acid triglyceride was used as the liquid medicine, the discharge amount was measured as a weight using a semi-microbalance, and then the discharge amount (µL) was calculated from the specific gravity of the liquid medicine (test temperature was room temperature). Specifically, the sample was discharged 10 times, and the total weight (mg) of the sample was measured to calculate the single discharge amount (µL).

The results are listed in Table 1.

### [Table 1]

**Table 1**

| | **Interval (second)** | **Single Dis charge Amount (µL)** | **Standard Deviation** |
|---|---|---|---|
| **Example 1** | 1 | 0.44 | 0.021 |
| | 5 | 0.46 | 0.028 |
| | 10 | 0.48 | 0.045 |
| | 20 | 0.50 | 0.015 |
| **Example 2** | 5 | 0.19 | 0.028 |
| | 10 | 0.17 | 0.005 |
| | 20 | 0.21 | 0.010 |

As listed in Table 1, the discharge amount of Example 1 was 1/80 or less as compared with the discharge amount from a normal eye drop bottle (about 40 µL to 50 µL), and was a sufficiently small amount. In Example 2 with the orifice, since discharge resistance is generated by the orifice, the discharge amount was smaller than that in Example 1 without the orifice.

As listed in Table 1, in each of Examples 1 and 2, the standard deviation of the single discharge amount (one droplet amount) was 0.05 or less even in the case where the sample was repeatedly discharged, that is, the accuracy was high. In Examples 1 and 2, when we changed the discharge interval, with repeatedly discharging, such as 1 second, 5 seconds, 10 seconds, and 20 seconds, the discharge amount tended to slightly increase, but the discharge amount was not at a level that causes a problem.

As described above, it was found that a small amount of the liquid medicine can be quantitatively administered with high accuracy. In addition, it was found that a small amount of the liquid medicine can be linearly discharged from the trajectory of the liquid medicine.

### EXPLANATION OF REFERENCE SIGNS

1, 500, 700: dosing device (liquid discharge device)
3, 511: striking member
5, 601: liquid medicine storage portion (liquid storage portion)
6: tube member
7: energizing member
8: discharge port
51, 570: striking portion
52: operation portion
100: liquid medicine
501: main body portion
502: liquid medicine cartridge (liquid cartridge)
510: case member
512: operation member (operation portion)
520, 521: case portion
550: liquid medicine passage hole (liquid passage hole)
551: case-side restriction groove
555: attachment portion
572: connection portion
580: operation surface portion
583: closing portion

## Claims

1. A liquid discharge device comprising:
a liquid storage portion (5, 601);
a tube member (6);
a striking portion (51, 570);
an operation portion (52); and
a discharge port (8) capable of discharging liquid,
wherein the tube member (6) has an end portion communicating with an inside of the liquid storage portion (5, 601), the tube member (6) delivering the liquid from the liquid storage portion (5, 601) to a discharge port side,
wherein the tube member (6) has an elastic region elastically deformable at least partly in a longitudinal direction, and
wherein when the operation portion (52) is operated, at least a part of the elastic region of the tube member (6) is struck with an impact force by the striking portion (51, 570) to allow the liquid in the tube member (6) to be pushed out and discharged from the discharge port (8);the liquid discharge device being **characterized in that**
the tube member (6) delivers the liquid from the liquid storage portion (5, 601) to the discharge port side by capillarity.

2. The liquid discharge device according to claim 1, wherein an entire region of the tube member (6) constitutes the elastic region.

3. The liquid discharge device according to any one of claim 1 or 2, wherein the elastic region is formed of at least one elastic material selected from the group consisting of polyethylene, polypropylene, silicone, fluororubber, polytetrafluoroethylene, nylon, polyethylene terephthalate, polybutylene terephthalate, polyethylene naphthalate, natural rubber, and synthetic rubber.

4. The liquid discharge device according to any one of claims 1 to 3, wherein a maximum inner diameter of the elastic region in the tube member (6) is 0.1 mm or more and 2 mm or less.

5. The liquid discharge device according to any one of claims 1 to 4, wherein a discharge amount of the liquid from the discharge port (8) when the operation portion (52) is operated once is 0.01 µL or more and 100 µL or less.

6. The liquid discharge device according to any one of claims 1 to 5, wherein an area of the tube member (6) struck by the striking portion (51, 570) is a range of 1 mm or more and 30 mm or less in a longitudinal direction.

7. The liquid discharge device according to any one of claims 1 to 6, wherein when the operation portion (52) is operated with the discharge port (8) facing a horizontal direction, the liquid is discharged from the discharge port (8) ,at least in a range of more than 0 cm and 0.5 cm or less, with an angle of the straight line connecting the liquid discharge port and an end point of the range being 5 degrees or less.

8. The liquid discharge device according to any one of claims 1 to 7, further comprising:
a main body portion (501); and
a liquid cartridge (502),
wherein the main body portion (501) includes the striking portion (51, 570) and the operation portion (52), and
wherein the liquid cartridge (502) includes the liquid storage portion (5, 601) and the tube member (6), the liquid cartridge (502) being attachable to and detachable from the main body portion (501).

9. The liquid discharge device according to any one of claims 1 to 8, further comprising:
a case portion (520, 521) accommodating at least a part of the tube member (6) so as to incorporate the discharge port (8) therein; and
a closing portion (583) configured to move or change a posture relative to the case portion (520, 521), following a movement of the operation portion (52),
the case portion (520, 521) having a liquid passage hole (550) at a position facing the discharge port (8),
wherein the liquid discharge device includes:
a closed state where the closing portion (583) closes the liquid passage hole (550); and
an open state where the closing portion (583) opens the liquid passage hole (550),
the closed state and the open state being changeable when the operation portion (52) is operated to move or to change a posture of the closing portion (583).

10. The liquid discharge device according to any one of claims 1 to 9, further comprising:
a case member (510); and
a striking member (3, 511) having the striking portion (51, 570),
wherein the striking member (3, 511) has a connection portion (572) supported in a cantilever manner by the case member (510),
wherein the elastic region of the tube member (6) is at a position higher than the connection portion (572), and
wherein when the operation portion (52) is operated, the striking portion (51, 570) is elastically deformed to allow the striking portion (51, 570) to strike at least a part of the elastic region by a restoring force.

11. A liquid discharge device according to any one of claims 1 to 10,
wherein the tube member (6) has a first end portion immersed in liquid inside the liquid storage portion (5, 601), and a second end communicating with an outside via the discharge port (8),
wherein the elastic region is at an intermediate portion in a longitudinal direction of the tube member (6), and has a maximum inner diameter of 0.1 mm or more and 2 mm or less, and
wherein when the operation portion (52) is operated, at least a part of the elastic region of the tube member (6) is struck with an impact force by the striking portion (51, 570) to allow the liquid inside the liquid storage portion (5, 601) to be pushed out and to be discharged from the discharge port (8).

12. A dosing device (1, 500, 700) using the liquid discharge device according to any one of claims 1 to 11, wherein the liquid is a liquid medicine (100).

## Patentansprüche

1. Flüssigkeitsausstoßvorrichtung, umfassend:
einen Flüssigkeitsspeicherabschnitt (5, 601);
ein Schlauchelement (6);
einen Schlagabschnitt (51, 570);
einen Betätigungsabschnitt (52); und
eine Ausstoßöffnung (8), die imstande ist, Flüssigkeit auszustoßen,
wobei das Schlauchelement (6) einen Endabschnitt aufweist, der mit einem Inneren des Flüssigkeitsspeicherabschnitts (5, 601) in Verbindung steht, wobei das Schlauchelement (6) die Flüssigkeit von dem Flüssigkeitsspeicherabschnitt (5, 601) zu einer Ausstoßöffnungsseite fördert,
wobei das Schlauchelement (6) einen elastischen Bereich aufweist, der mindestens teilweise in einer Längsrichtung elastisch verformbar ist, und
wobei, wenn der Betätigungsabschnitt (52) betätigt wird, mindestens ein Teil des elastischen Bereichs des Schlauchelements (6) von dem Schlagabschnitt (51, 570) mit einer Schlagkraft geschlagen wird, sodass die Flüssigkeit in dem Schlauchelement (6) herausgedrückt und aus der Ausstoßöffnung (8) ausgestoßen werden kann; wobei die Flüssigkeitsausstoßvorrichtung **dadurch gekennzeichnet ist, dass**
das Schlauchelement (6) die Flüssigkeit von dem Flüssigkeitsspeicherabschnitt (5, 601) durch Kapillarwirkung zu der Ausstoßöffnungsseite fördert.

2. Flüssigkeitsausstoßvorrichtung nach Anspruch 1, wobei ein gesamter Bereich des Schlauchelements (6) den elastischen Bereich bildet.

3. Flüssigkeitsausstoßvorrichtung nach einem der Ansprüche 1 oder 2, wobei der elastische Bereich aus mindestens einem elastischen Material gebildet ist, ausgewählt aus der Gruppe bestehend aus Polyethylen, Polypropylen, Silikon, Fluorkautschuk, Polytetrafluorethylen, Nylon, Polyethylenterephthalat, Polybutylenterephthalat, Polyethylennaphthalat, Naturkautschuk und Synthesekautschuk.

4. Flüssigkeitsausstoßvorrichtung nach einem der Ansprüche 1 bis 3, wobei ein maximaler Innendurchmesser des elastischen Bereichs in dem Schlauchelement (6) 0,1 mm oder mehr und 2 mm oder weniger beträgt.

5. Flüssigkeitsausstoßvorrichtung nach einem der Ansprüche 1 bis 4, wobei eine Ausstoßmenge der Flüssigkeit aus der Ausstoßöffnung (8), wenn der Betätigungsabschnitt (52) einmal betätigt wird, 0,01 µL oder mehr und 100 µL oder weniger beträgt.

6. Flüssigkeitsausstoßvorrichtung nach einem der Ansprüche 1 bis 5, wobei ein Bereich des Schlauchelements (6), der von dem Schlagabschnitt (51, 570) geschlagen wird, in einer Längsrichtung ein Bereich von 1 mm oder mehr und 30 mm oder weniger ist.

7. Flüssigkeitsausstoßvorrichtung nach einem der Ansprüche 1 bis 6, wobei, wenn der Betätigungsabschnitt (52) betätigt wird, während die Ausstoßöffnung (8) in eine horizontale Richtung weist, die Flüssigkeit aus der Ausstoßöffnung (8) mindestens in einem Bereich von mehr als 0 cm und 0,5 cm oder weniger ausgestoßen wird, wobei ein Winkel der geraden Linie, die die Ausstoßöffnung und einen Endpunkt des Bereichs verbindet, 5 Grad oder weniger beträgt.

8. Flüssigkeitsausstoßvorrichtung nach einem der Ansprüche 1 bis 7, weiter umfassend:
einen Hauptkörperabschnitt (501); und
eine Flüssigkeitskartusche (502),
wobei der Hauptkörperabschnitt (501) den Schlagabschnitt (51, 570) und den Betätigungsabschnitt (52) einschließt, und
wobei die Flüssigkeitskartusche (502) den Flüssigkeitsspeicherabschnitt (5, 601) und das Schlauchelement (6) einschließt, wobei die Flüssigkeitskartusche (502) an dem Hauptkörperabschnitt (501) anbringbar und von diesem abnehmbar ist.

9. Flüssigkeitsausstoßvorrichtung nach einem der Ansprüche 1 bis 8, weiter umfassend:
einen Gehäuseabschnitt (520, 521), der mindestens einen Teil des Schlauchelements (6) aufnimmt, um die Ausstoßöffnung (8) darin aufzunehmen; und
einen Verschlussabschnitt (583), der dazu ausgebildet ist, sich relativ zu dem Gehäuseabschnitt (520, 521) zu bewegen oder seine Lage zu ändern, einer Bewegung des Betätigungsabschnitts (52) folgend,
wobei der Gehäuseabschnitt (520, 521) an einer Position, die der Ausstoßöffnung (8) gegenüberliegt, ein Flüssigkeitsdurchgangsloch (550) aufweist,
wobei die Flüssigkeitsausstoßvorrichtung Folgendes einschließt:
einen geschlossenen Zustand, in dem der Verschlussabschnitt (583) das Flüssigkeitsdurchgangsloch (550) verschließt; und
einen offenen Zustand, in dem der Verschlussabschnitt (583) das Flüssigkeitsdurchgangsloch (550) öffnet,
wobei der geschlossene Zustand und der offene Zustand veränderbar sind, wenn der Betätigungsabschnitt (52) betätigt wird, um den Verschlussabschnitt (583) zu bewegen oder seine Lage zu ändern.

10. Flüssigkeitsausstoßvorrichtung nach einem der Ansprüche 1 bis 9, weiter umfassend:
ein Gehäuseelement (510); und
ein Schlagelement (3, 511), das den Schlagabschnitt (51, 570) aufweist,
wobei das Schlagelement (3, 511) einen Verbindungsabschnitt (572) aufweist, der von dem Gehäuseelement (510) kragarmartig getragen ist,
wobei der elastische Bereich des Schlauchelements (6) an einer Position höher als der Verbindungsabschnitt (572) ist, und
wobei, wenn der Betätigungsabschnitt (52) betätigt wird, der Schlagabschnitt (51, 570) elastisch verformt wird, sodass der Schlagabschnitt (51, 570) mindestens einen Teil des elastischen Bereichs durch eine Rückstellkraft schlagen kann.

11. Flüssigkeitsausstoßvorrichtung nach einem der Ansprüche 1 bis 10,
wobei das Schlauchelement (6) einen ersten Endabschnitt aufweist, der in Flüssigkeit innerhalb des Flüssigkeitsspeicherabschnitts (5, 601) eingetaucht ist, und ein zweites Ende, das über die Ausstoßöffnung (8) mit einem Außenbereich in Verbindung steht,
wobei der elastische Bereich an einem Zwischenabschnitt in einer Längsrichtung des Schlauchelements (6) angeordnet ist und einen maximalen Innendurchmesser von 0,1 mm oder mehr und 2 mm oder weniger aufweist, und
wobei, wenn der Betätigungsabschnitt (52) betätigt wird, mindestens ein Teil des elastischen Bereichs des Schlauchelements (6) von dem Schlagabschnitt (51, 570) mit einer Schlagkraft geschlagen wird, sodass die Flüssigkeit innerhalb des Flüssigkeitsspeicherabschnitts (5, 601) herausgedrückt und aus der Ausstoßöffnung (8) ausgestoßen werden kann.

12. Dosiervorrichtung (1, 500, 700) unter Verwendung der Flüssigkeitsausstoßvorrichtung nach einem der Ansprüche 1 bis 11, wobei die Flüssigkeit ein flüssiges Arzneimittel (100) ist.

## Revendications

1. Dispositif d'évacuation de liquide comprenant :
une portion de stockage de liquide (5, 601) ;
un élément tubulaire (6) ;
une portion de percussion (51, 570) ;
une portion d'actionnement (52) ; et
un orifice d'évacuation (8) apte à évacuer du liquide,
dans lequel l'élément tubulaire (6) présente une portion d'extrémité en communication avec un intérieur de la portion de stockage de liquide (5, 601), l'élément tubulaire (6) acheminant le liquide depuis la portion de stockage de liquide (5, 601) vers un côté de l'orifice d'évacuation,
dans lequel l'élément tubulaire (6) présente une région élastique élastiquement déformable au moins en partie dans une direction longitudinale, et
dans lequel, lorsque la portion d'actionnement (52) est actionnée, au moins une partie de la région élastique de l'élément tubulaire (6) est percutée avec une force d'impact par la portion de percussion (51, 570) pour permettre au liquide dans l'élément tubulaire (6) d'être poussé vers l'extérieur et évacué depuis l'orifice d'évacuation (8) ; le dispositif d'évacuation de liquide étant **caractérisé en ce que**
l'élément tubulaire (6) achemine le liquide depuis la portion de stockage de liquide (5, 601) vers le côté de l'orifice d'évacuation par capillarité.

2. Dispositif d'évacuation de liquide selon la revendication 1, dans lequel une région entière de l'élément tubulaire (6) constitue la région élastique.

3. Dispositif d'évacuation de liquide selon l'une quelconque de la revendication 1 ou de la revendication 2, dans lequel la région élastique est formée d'au moins un matériau élastique sélectionné à partir du groupe constitué du polyéthylène, du polypropylène, du silicone, du caoutchouc fluoré, du polytétrafluoroéthylène, du nylon, du polyéthylène téréphtalate, du polybutylène téréphtalate, du polyéthylène naphtalate, du caoutchouc naturel et du caoutchouc synthétique.

4. Dispositif d'évacuation de liquide selon l'une quelconque des revendications 1 à 3, dans lequel un diamètre interne maximal de la région élastique dans l'élément tubulaire (6) est de 0,1 mm ou plus et de 2 mm ou moins.

5. Dispositif d'évacuation de liquide selon l'une quelconque des revendications 1 à 4, dans lequel une quantité évacuée du liquide depuis l'orifice d'évacuation (8) lorsque la portion d'actionnement (52) est actionnée une fois est de 0,01 µL ou plus et de 100 µL ou moins.

6. Dispositif d'évacuation de liquide selon l'une quelconque des revendications 1 à 5, dans lequel une zone de l'élément tubulaire (6) percutée par la portion de percussion (51, 570) est une plage de 1 mm ou plus et de 30 mm ou moins dans une direction longitudinale.

7. Dispositif d'évacuation de liquide selon l'une quelconque des revendications 1 à 6, dans lequel, lorsque la portion d'actionnement (52) est actionnée avec l'orifice d'évacuation (8) faisant face à une direction horizontale, le liquide est évacué depuis l'orifice d'évacuation (8), au moins dans une plage de plus de 0 cm et de 0,5 cm ou moins, un angle de la ligne droite reliant l'orifice d'évacuation de liquide et un point d'extrémité de la plage étant de 5 degrés ou moins.

8. Dispositif d'évacuation de liquide selon l'une quelconque des revendications 1 à 7, comprenant en outre :
une portion de corps principal (501) ; et
une cartouche de liquide (502),
dans lequel la portion de corps principal (501) inclut la portion de percussion (51, 570) et la portion d'actionnement (52), et
dans lequel la cartouche de liquide (502) inclut la portion de stockage de liquide (5, 601) et l'élément tubulaire (6), la cartouche de liquide (502) pouvant être fixée à et détachée de la portion de corps principal (501).

9. Dispositif d'évacuation de liquide selon l'une quelconque des revendications 1 à 8, comprenant en outre :
une portion de boîtier (520, 521) logeant au moins une partie de l'élément tubulaire (6) de façon à y incorporer l'orifice d'évacuation (8) ; et
une portion de fermeture (583) configurée pour se déplacer ou changer de posture par rapport à la portion de boîtier (520, 521), à la suite d'un mouvement de la portion d'actionnement (52),
la portion de boîtier (520, 521) présentant un trou de passage de liquide (550) à une position faisant face à l'orifice d'évacuation (8),
dans lequel le dispositif d'évacuation de liquide inclut :
un état fermé où la portion de fermeture (583) ferme le trou de passage de liquide (550) ; et
un état ouvert où la portion de fermeture (583) ouvre le trou de passage de liquide (550),
l'état fermé et l'état ouvert pouvant être changés lorsque la portion d'actionnement (52) est actionnée pour déplacer la portion de fermeture (583) ou lui faire changer de posture.

10. Dispositif d'évacuation de liquide selon l'une quelconque des revendications 1 à 9, comprenant en outre :
un élément de boîtier (510) ; et
un élément de percussion (3, 511) présentant la portion de percussion (51, 570),
dans lequel l'élément de percussion (3, 511) présente une portion de liaison (572) supportée en porte-à-faux par l'élément de boîtier (510),
dans lequel la région élastique de l'élément tubulaire (6) est à une position plus haute que la portion de liaison (572), et
dans lequel, lorsque la portion d'actionnement (52) est actionnée, la portion de percussion (51, 570) est élastiquement déformée pour permettre à la portion de percussion (51, 570) de percuter au moins une partie de la région élastique par une force de rappel.

11. Dispositif d'évacuation de liquide selon l'une quelconque des revendications 1 à 10,
dans lequel l'élément tubulaire (6) présente une première portion d'extrémité immergée dans du liquide à l'intérieur de la portion de stockage de liquide (5, 601), et une seconde extrémité en communication avec un extérieur via l'orifice d'évacuation (8),
dans lequel la région élastique est à une portion intermédiaire dans une direction longitudinale de l'élément tubulaire (6), et présente un diamètre interne maximal de 0,1 mm ou plus et de 2 mm ou moins, et
dans lequel, lorsque la portion d'actionnement (52) est actionnée, au moins une partie de la région élastique de l'élément tubulaire (6) est percutée avec une force d'impact par la portion de percussion (51, 570) pour permettre au liquide à l'intérieur de la portion de stockage de liquide (5, 601) d'être poussé vers l'extérieur et d'être évacué depuis l'orifice d'évacuation (8).

12. Dispositif de dosage (1, 500, 700) utilisant le dispositif d'évacuation de liquide selon l'une quelconque des revendications 1 à 11, dans lequel le liquide est un médicament liquide (100).
